# EUROPÄISCHE PATENTSCHRIFT

(11) **EP 0 706 527 B1**
(45) Veröffentlichungstag und Bekanntmachung des Hinweises auf die Patenterteilung: **14.11.2001**
(21) Anmeldenummer: 94920958.9
(22) Anmeldetag: 20.06.1994
(51) Int. Cl.: C07D 495/10, C07D 491/107, C07D 471/10, C07F 9/6544, C07D 333/38, C07D 335/02, C07D 309/14, C07D 211/66, A01N 43/90

(54) **SUBSTITUIERTE SPIROHETEROCYCLISCHE 1H-3-ARYL-PYRROLIDIN-2,4-DION-DERIVATE, VERFAHREN ZU DEREN HERSTELLUNG UND DEREN VERWENDUNG ALS SCHÄDLINGSBEKÄMPFUNGSMITTEL**
SUBSTITUTED SPIROHETEROCYCLIC 1H-3-ARYLPYRROLIDINE-2,4-DIONE DERIVATIVES, METHODS OF PREPARING THEM AND THEIR USE AS PEST-CONTROL AGENTS
DERIVES SUBSTITUES SPIRO-HETEROCYCLIQUES DE LA 1H-3-ARYLPYRROLIDINE-2,4-DIONE, LEUR PROCEDE DE PREPARATION ET LEUR UTILISATION COMME INSECTICIDES

(30) Priorität: 02.07.1993 DE 4322052; 07.01.1994 DE 4400223; 02.05.1994 DE 4415334
(43) Veröffentlichungstag der Anmeldung: 17.04.1996
(73) Patentinhaber: BAYER AG, 51368 Leverkusen (DE)
(72) Erfinder: FISCHER, Reiner, D-40789 Monheim (DE); BRETSCHNEIDER, Thomas, D-53721 Siegburg (DE); KRÜGER, Bernd-Wieland, D-51467 Bergisch Gladbach (DE); SANTEL, Hans-Joachim, D-51371 Leverkusen (DE); DOLLINGER, Markus, D-51381 Leverkusen (DE); ERDELEN, Christoph, D-42799 Leichlingen (DE); WACHENDORFF-NEUMANN, Ulrike, D-40789 Monheim (DE)
(86) Internationale Anmeldenummer: EP9401997
(87) Internationale Veröffentlichungsnummer: WO9501358

(56) Entgegenhaltungen:
- EP-A- 0 456 063
- EP-A- 0 521 334
- EP-A- 0 595 130
- EP-A- 0 596 298
- EP-A- 0 613 884

## Beschreibung

Die Erfindung betrifft neue substituierte spirocyclische 1H-3-Aryl-pyrrolidin-2,4-dion-Derivate, mehrere Verfahren zu ihrer Herstellung und ihre Verwendung als Schädlingsbekämpfungsmittel und als Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985 1095) synthetisiert. Eine biologische Wirksamkeit dieser Verbindungen wurde nicht beschrieben. Weiterhin wurden 5-Vinyl-tetramsäuren mit pharmazeutischen Eigenschaften beschrieben (GB-A 22 66 888).

In EP-A 0 262 399 werden 3-Aryl-pyrrolidin-2,4-dione offenbart, von denen jedoch keine herbizide, insektizide oder akarizide Wirkung bekannt geworden ist. Die herbizide, insektizide oder akarizide Wirkung ist bekannt von unsubstituierten, bicyclischen 3-Aryl-pyrrolidin-2,4-dion-Derivaten (EP-A 355 599) und (EP 415 211) sowie von substituierten mono-cyclischen 3-Aryl-pyrrolidin-2,4-dion-Derivaten (EP-A 377 893), (EP 442 077), (EP 497 127) und substituierten bicyclischen 3-Aryl-pyrrolidon-Derivaten (EP 501 129).

Weiterhin bekannt sind polycyclische 3-Arylpyrrolidin-2,4-dion-Derivate (EP 442 073) sowie lH-3-Arylpyrrolidin-dion-Derivate (EP 456 063) und (EP 521 334).

Es wurden nun neue substituierte spirocyclische lH-3-Aryl-pyrrolidin-2,4-dion-derivate der Formel (I) 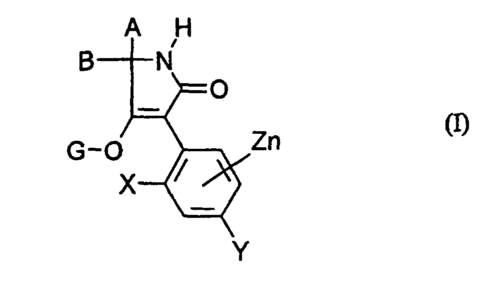 gefunden, in welcher
- A, B: und das Kohlenstoffatom, an das sie gebunden sind, für einen 5- bis 6-gliedrigen Spirocyclus stehen, der Sauerstoff oder Schwefel enthält, der unsubstituiert ist,
- X: für C₁-C₆-Alkyl, Halogen oder C₁-C₆-Alkoxy steht,
- Y: für Wasserstoff, C₁-C₆-Alkyl, Halogen, C₁-C₆-Alkoxy oder C₁-C₃-Halogenalkyl steht,
- Z: für C₁-C₆-Alkyl, Halogen oder C₁-C₆-Alkoxy steht,
- G: für die Gruppen 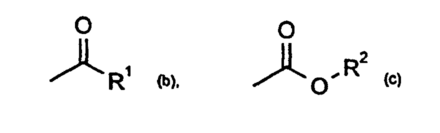 steht,
in welchen
R¹ für jeweils gegebenenfalls durch Halogen substituiertes C₁-C₂₀-Alkyl, C₂-C₁₀-Alkenyl, C₁-C₈-Alkoxy-C₁-C₈-alkyl, C₁-C₈-Alkyl-thio-C₁-C₈-alkyl, C₁-C₈-Polyalkoxy-C₁-C₈-alkyl oder Cycloalkyl mit 3 bis 8 Ringatomen, das durch Sauerstoff-und/oder Schwefelatome unterbrochen und durch C₁-C₆-Alkyl substituiert sein kann,
für gegebenenfalls durch Halogen, Nitro, C₁-C₆-Alkyl, C₁-C₆-Alkoxy, C₁-C₆-Halogenalkyl oder C₁-C₆-Halogenalkoxy substituiertes Phenyl,
für gegebenenfalls durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl
für gegebenenfalls durch Halogen und/oder C₁-C₆-Alkyl substituiertes Hetaryl,
für gegebenenfalls durch Halogen und/oder C₁-C₆-Alkyl substituiertes Phenoxy-C₁-C₆-alkyl oder
für gegebenenfalls durch Halogen, Amino und/oder C₁-C₆-Alkyl substituiertes Hetaryloxy-C₁-C₆-alkyl steht,
R² für jeweils gegebenenfalls durch Halogen substituiertes C₁-C₂₀-Alkyl, C₃-C₁₀-Alkenyl, C₁-C₈-Alkoxy-C₂-C₈-alkyl, C₁-C₈-Polyalkoxy-C₂-C₈-alkyl
für gegebenenfalls durch Halogen oder C₁-C₆-Alkyl substituiertes C₃-C₆-Cycloalkyl oder
für jeweils gegebenenfalls durch Halogen, Nitro, C₁-C₆-Alkyl, C₁-C₆-Alkoxy oder C₁-C₆-Halogenalkyl substituiertes Phenyl oder Benzyl steht und
- n: für eine Zahl von 0 bis 3 steht.

Unter Einbeziehung der verschiedenen Bedeutungen (b) und (c) der Gruppe G der allgemeinen Formel (I) ergeben sich folgende hauptsächlichen Shukturen (Ib) bis (Ic): 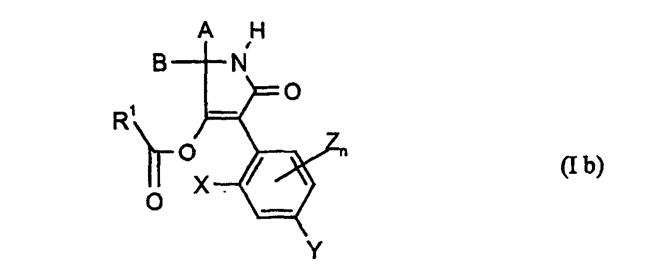 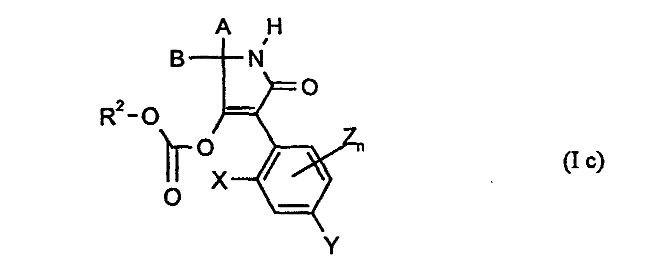 worin

A, B, X, Y, Z, R¹, R² und n die oben angegebenen Bedeutungen besitzen.

Aufgrund eines oder mehrerer Chiralitätszentren, fallen die Verbindungen der Formel (Ib) - (Ic) im allgemeinen als Stereoisomerengemisch an. Sie können sowohl in Form ihrer Diastereomerengemische als auch als reine Diastereomere oder Enantiomere verwendet werden.

Weiterhin wurde gefunden, daß man die neuen substituierten 1H-3-Aryl-pyrrolidin-2,4-dion-Derivate der Formel (I) nach einem der im folgenden beschriebenen Verfahren erhält.

(B) Man erhält Verbindungen der Formel (Ib) 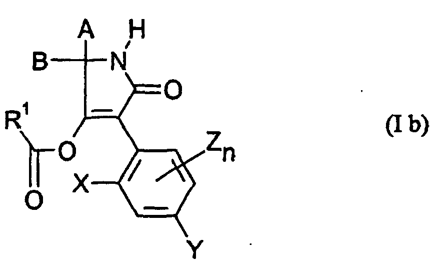 in welcher
A, B, X, Y, Z, R¹ und n die oben angegebene Bedeutung haben, wenn man Verbindungen der Formel (Ia), 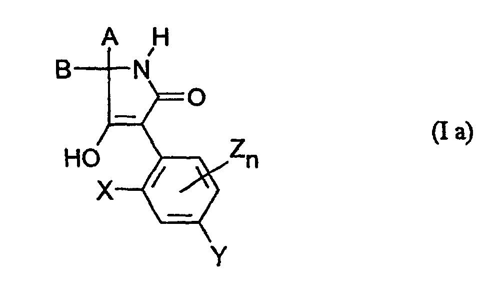 in welcher
A, B, X, Y, Z und n die oben angegebene Bedeutung haben,
α) mit Säurehalogeniden der Formel (III) 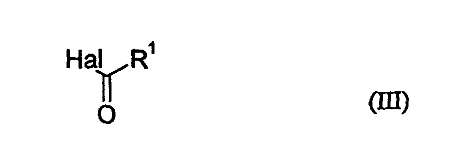 in welcher
   - R¹: die oben angegebene Bedeutung hat und
   - Hal: für Halogen, insbesondere Chlor und Brom steht,
   gegebenenfalls in Gegenwart eines Verdünnungsmittels und gegebenenfalls in Gegenwart eines Säurebindemittels umsetzt
   oder
β) mit Carbonsäureanhydriden der Formel (IV)

   R¹-CO-O-CO-R¹ (IV)

   in welcher
   - R¹: die oben angegebene Bedeutung hat,
   gegebenenfalls in Gegenwart eines Verdünnungsmittels und gegebenenfalls in Gegenwart eines Säurebindemittels, umsetzt;
oder

(C) man erhält Verbindungen der Formel (Ic-1) 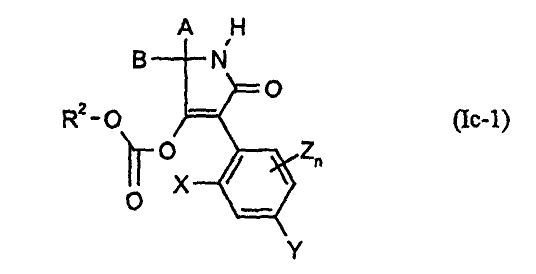 in welcher
A, B, X, Y, Z, R² und n die oben angegebene Bedeutung haben, wenn man Verbindungen der Formel (Ia) 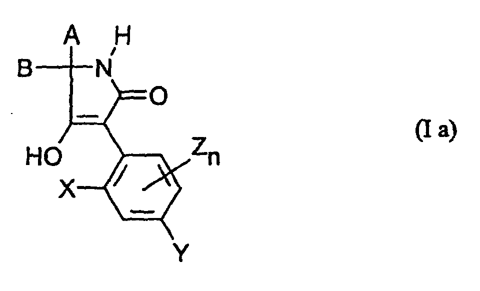 in welcher
A, B, X, Y, Z und n die oben angegebene Bedeutung haben, mit Chlorameisensäureester oder Chlorameisensäurethiolester der Formel (V)

R²-O-CO-Cl (V)

in welcher
- R²: die oben angegebene Bedeutung haben, gegebenenfalls in Gegenwart eines Verdünnungsmittels und gegebenenfalls in Gegenwart eines Säurebindemittels umsetzt.

Weiterhin wurde gefunden, daß sich die neuen 1H-3-Arylpyrrolidin-2,4-dion-Derivate der Formel (I) durch hervorragende insektizide, akarizide und herbizide Wirkungen auszeichnen.

Bevorzugt sind Verbindungen der Formel (I), in der
- A,B: und das Kohlenstoffatom, an das sie gebunden sind, für einen 5- bis 6-gliedrigen Spirocyclus stehen, der Sauerstoff oder Schwefel enthält, der unsubstituiert ist,
- X: für C₁-C₄-Alkyl, Halogen oder C₁-C₄-Alkoxy steht,
- Y: für Wasserstoff, C₁-C₄-Alkyl, Halogen, C₁-C₄-Alkoxy oder C₁-C₂-Halogenalkyl steht,
- Z: für C₁-C₄-Alkyl, Halogen oder C₁-C₄-Alkoxy steht,
- G: für die Gruppen 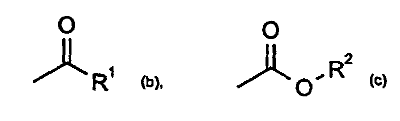 steht, in welchen
R¹ für jeweils gegebenenfalls durch Halogen substituiertes C₁-C₁₆-Alkyl, C₂-C₈-Alkenyl, C₁-C₆-Alkoxy-C₁-C₆-alkyl, C₁-C₆-Alkyl-thio-C₁-C₆-alkyl, C₁-C₆-Polyalkoxy-C₁-C₆-alkyl oder Cycloalkyl mit 3 bis 7 Ringatomen, das durch 1-2 Sauerstoff- und/oder Schwefelatome unterbrochen und durch C₁-C₄-Alkyl substituiert sein kann,
für gegebenenfalls durch Halogen, Nitro, C₁-C₄-Alkyl, C₁-C₄-Alkoxy, C₁-C₃-Halogenalkyl oder C₁-C₃-Halogenalkoxy substituiertes Phenyl,
für gegebenenfalls durch Halogen, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom und/oder C₁-C₄-Alkyl substituiertes Furanyl, Thienyl, Pyridyl, Pyrimidyl, Thiazolyl oder Pyrazolyl,
für gegebenenfalls durch Fluor, Chlor, Brom und/oder C₁-C₄-Alkyl substituiertes Phenoxy-C₁-C₅-alkyl oder
für jeweils gegebenenfalls durch Fluor, Chlor, Brom, Amino und/oder C₁-C₄-Alkyl substituiertes Pyridyloxy-C₁-C₆-alkyl, Pyrimidinyloxy-C₁-C₆-alkyl oder Thiazolyloxy-C₁-C₆-alkyl steht,
R² für jeweils gegebenenfalls durch Halogen substituiertes C₁-C₁₆-Alkyl, C₃-C₈-Alkenyl, C₁-C₆-Alkoxy-C₂-C₆-alkyl, C₁-C₆-Polyalkoxy-C₂-C₆-alkyl,
für gegebenenfalls durch Fluor, Chlor oder C₁-C₄-Alkyl substituiertes C₃-C₆-Cycloalkyl oder
für jeweils gegebenenfalls durch Halogen, Nitro, C₁-C₄-Alkyl, C₁-C₃-Alkoxy oder C₁-C₃-Halogenalkyl substituiertes Phenyl oder Benzyl steht und
- n: für eine Zahl von 0 bis 2 steht.

Besonders bevorzugt sind Verbindungen der Formel (I), in welcher
- A, B: und das Kohlenstoffatom, an das sie gebunden sind, für einen 5- bis 6-gliedrigen Spirocyclus stehen, der Sauerstoff oder Schwefel enthält, der unsubstituiert ist,
- X: für Methyl, Ethyl, Propyl, 2-Propyl, Fluor, Chlor, Brom, Methoxy oder Ethoxy steht,
- Y: für Wasserstoff, Methyl, Ethyl, Propyl, i-Propyl, Butyl, i-Butyl, tert.-Butyl, Fluor, Chlor, Brom, Methoxy, Ethoxy oder Trifluormethyl steht,
- Z: für Methyl, Ethyl, Propyl, i-Propyl, Butyl, i-Butyl, tert.-Butyl, Fluor, Chlor, Brom, Methoxy oder Ethoxy steht,
- G: für die Gruppen 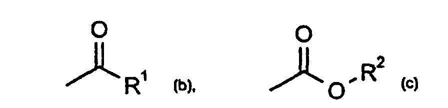 steht, in welchen
R¹ für jeweils gegebenenfalls durch Fluor oder Chlor substituiertes C₁-C₁₄-Alkyl, C₂-C₆-Alkenyl, C₁-C₄-Alkoxy-C₁-C₆-alkyl, C₁-C₄-Alkylthio-C₁-C₆-alkyl, C₁-C₄-Polyalkoxy-C₁-C₄-alkyl oder Cycloalkyl mit 3 bis 6 Ringatomen, das durch 1 bis 2 Sauerstoff- und/oder Schwefelatome unterbrochen und durch Methyl oder Ethyl substituiert sein kann,
für gegebenenfalls durch Fluor, Chlor, Brom, Methyl, Ethyl, Propyl, i-Propyl, Methoxy, Ethoxy, Trifluormethyl, Trifluormethoxy oder Nitro substituiertes Phenyl,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl,
für jeweils gegebenenfalls durch Fluor, Chlor, Brom, Methyl, Ethyl substituiertes Furanyl, Thienyl, Pyridyl, Pyrimidyl, Thiazolyl oder Pyrazolyl,
für gegebenenfalls durch Fluor, Chlor, Methyl, Ethyl substituiertes Phenoxy-C₁-C₄-alkyl oder
für jeweils gegebenenfalls durch Fluor, Chlor, Amino, Methyl, Ethyl substituiertes Pyridyloxy-C₁-C₄-alkyl, Pyrimidinyloxy-C₁-C₄-alkyl oder Thiazolyloxy-C₁-C₄-alkyl steht,
R² für jeweils gegebenenfalls durch Fluor oder Chlor substituiertes C₁-C₁₄-Alkyl, C₃-C₆-Alkenyl, C₁-C₄-Alkoxy-C₂-C₆-alkyl, C₁-C₄-Polyalkoxy-C₂-C₆-alkyl, für gegebenenfalls durch Fluor, Chlor, Methyl oder Ethyl substituiertes C₃-C₆-Cycloalkyl
oder für jeweils gegebenenfalls durch Fluor, Chlor, Nitro, Methyl, Ethyl, Propyl, i-Propyl, Methoxy, Ethoxy, Trifluormethyl substituiertes Phenyl oder Benzyl steht und
- n: für 0 oder 1 steht.

Aufgrund eines oder mehrerer Chiralitätszentren fallen die Verbindungen der Formel (Ib) bis (Ic) im allgemeinen als Stereoisomerengemische an. Sie können sowohl in

Form ihrer Diastereomerengemische als auch als reine Diastereomere oder Enantiomere verwendet werden.

Verwendet man gemäß Verfahren (B) (Variante a) 3-(2,4,6-Trimethylphenyl)-5,5-(ethylenthioethylen)-pyrrolidin-2,4-dion und Pivaloylchlorid als Ausgangsstoffe, so kann der Verlauf des erfindungsgemäßen Verfahrens durch folgendes Reaktionsschema wiedergegeben werden:

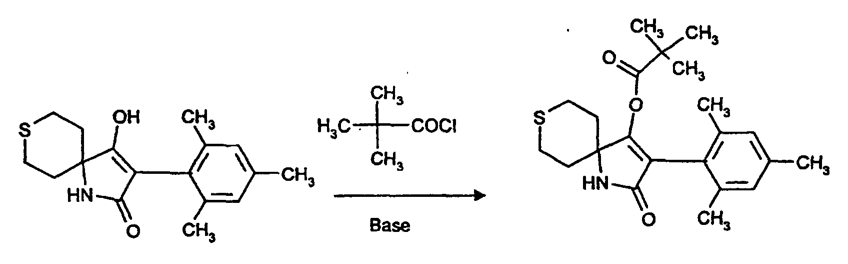

Verwendet man gemäß Verfahren B (Variante β) 3-(2,4,6-Trimethylphenyl)-5,5-ethylenthiomethylen-pyrrolidin-2,4-dion und Acetanhydrid als Ausgangsverbindungen, so kann der Verlauf des erfindungsgemäßen Verfahrens durch folgendes Reaktionsschema wiedergegeben werden:

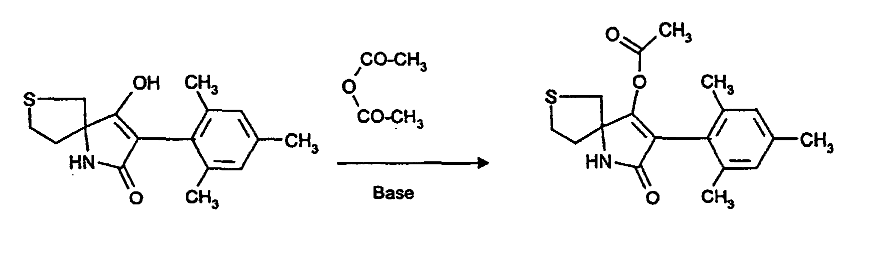

Verwendet man gemäß Verfahren (C) 3-(2,4,6-Trimethylphenyl)-5,5-ethylenthiomethylen-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden.

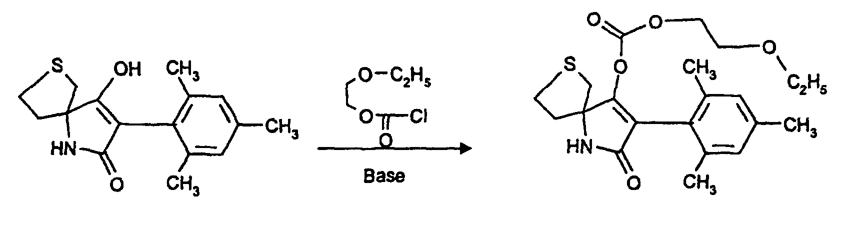

Die durch Heteroatome unterbrochenen cyclischen Aminocarbonsäuren der Formel (XIVa), in welcher A und B die oben angegebene Bedeutung haben, sind im allgemeinen nach der Bucherer-Bergs-Reaktion oder nach der Strecker-Synthese erhältlich.

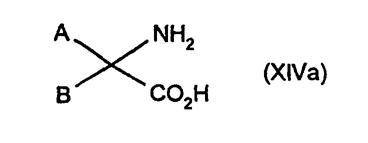

(L. Munday, J. Chem. Soc. 4372 (1961); J.T. Eward, C. Jitrangeri, Can. J. Chem. 53, 3339 (1975).

Die zur Durchführung der erfindungsgemäßen Verfahren (B) und (C) außerdem als Ausgangsstoffe benötigten Säurehalogenide der Formel (III), Carbonsäureanhydride der Formel (IV), Chlorameisensäureester (V) sind allgemein bekannte Verbindungen der organischen bzw. anorganischen Chemie.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Ba) bei Verwendung der Säurehalogenide alle gegenüber diesen Verbindungen inerten Solventien eingesetzt werden. Vorzuasweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecen (DBU), Diazabicyclononen (DEN), Hünig-Base und N,N-Dimethyl-anilin, ferner Erdalkalimetalloxide, wie Magnesium- und Calciumoxid, außerdem Alkali- und Erdalkalimetall-carbonate, wie Natriumcarbonat, Kaliumcarbonat und Calciumcarbonat sowie Alkalihydroxide wie Natriumhydroxid und Kaliumhydroxid.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Verwendet man die entsprechenden Chlorameisensäureester
so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) bei Verwendung der Chlorameisensäureester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) zur Bekämpfung von pflanzenschädigenden Milnben, wie beispielsweise gegen die gemeine Spinnmilbe oder die Bohnenspinnmilbe (Tetranychus urticae) einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Grattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Ncotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachauflaufverfahren. Sie können beispielsweise in Baumwolle oder Zuckerrüben mit sehr gutem Erfolg zur Bekämpfung von Schadgräsern eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. l-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (META-MITRON) zur Unkrautbekämpfung in Zuckerrüben und 4-Amino-6-(1,1-dimethyl-ethyl)-3-methylthio-1,2,4-triazin-5(4H)-on (METRIBUZIN) zur Unkrautbekämpfung in Sojabohnen, in Frage. Weiterhin kommen 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N- [(4-methoxy-6-methyl-1,3,5-triazin-2-yl)-amino]-carbonyl -benzolsulfonamid (CHLORSULFURON); 2-[4-(2,4-Dichlorphenoxy)-phenoxy]-propionsäure, deren Methyl- oder deren Ethylester (DICLOFOPMETHYL); 4-Amino-6-t-butyl-3-ethylthio-1,2,4-triazin-5(4H)-on (ETHIOZIN); 2- 4-[(6-Chlor-2-benzoxazolyl)-oxy]-phenoxy -propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yl-oxy)-acetanilid (MEFENACET); 2- [[((4-Methoxy-6-methyl-1,3,5-triazin-2-yl)-amino)-carbonyl]-amino]-sulfonyl -benzoesäure oder deren (METSULFURON); N-(1-Ethylpropyl)-3,4-dimethyl-2,6-dinitroanilin (PENDIMETHALIN); 0-(6-Chlor-3-phenylpyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure- (THIAMETURON) in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

### Genannt seien die folgenden Verbindungen:

Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zeta-methrin, Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb, Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion, Buprofezin, Chlorfluazuron, Diflubenzuron, Flucycloxuron, Flufenoxuron, Hexaflumuron, Pyriproxifen, Tebufenozide, Teflubenzuron, Triflumuron, Imidacloprid, Niteapyram, N-[(6-Chloro-3-pyridinyl)methyl]-N'-cyano-N-methyl-ethanimidamid (NI-25), Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron, Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox, Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3-carbonitril

### (AC 303630).

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Herstellungsbeispiele

### Beispiel I b - 1

5,8 g der Verbindung des Beispiels I a-1 werden in 70 ml trockenem Methylenchlorid mit 2,8 ml Triethylamin versetzt und bei 0 bis 10°C 1,5 ml Acetylchlorid in 5 ml trockenem Methylenchlorid zugegeben. Die Reaktionslösung wird zweimal mit 200 ml 0,5 N Natronlauge gewaschen, über Magnesiumsulfat getrocknet und das Lösungsmittel abdestilliert. Es bleiben 1,7 g (26 % d. Th.) Fp. 211°C.

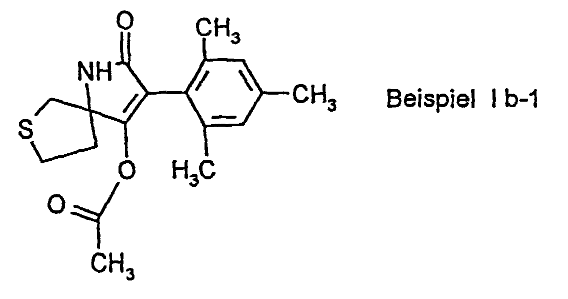

Analog werden die folgenden Verbindungen erhalten:

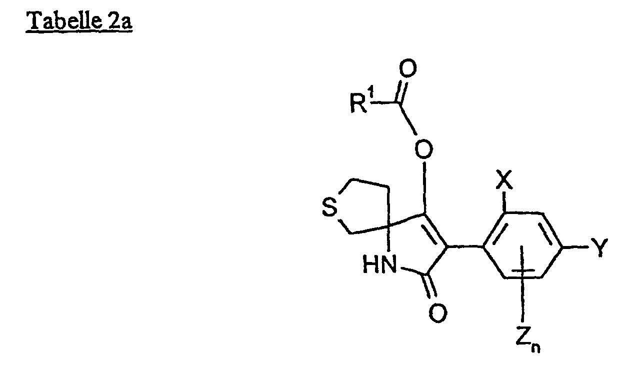

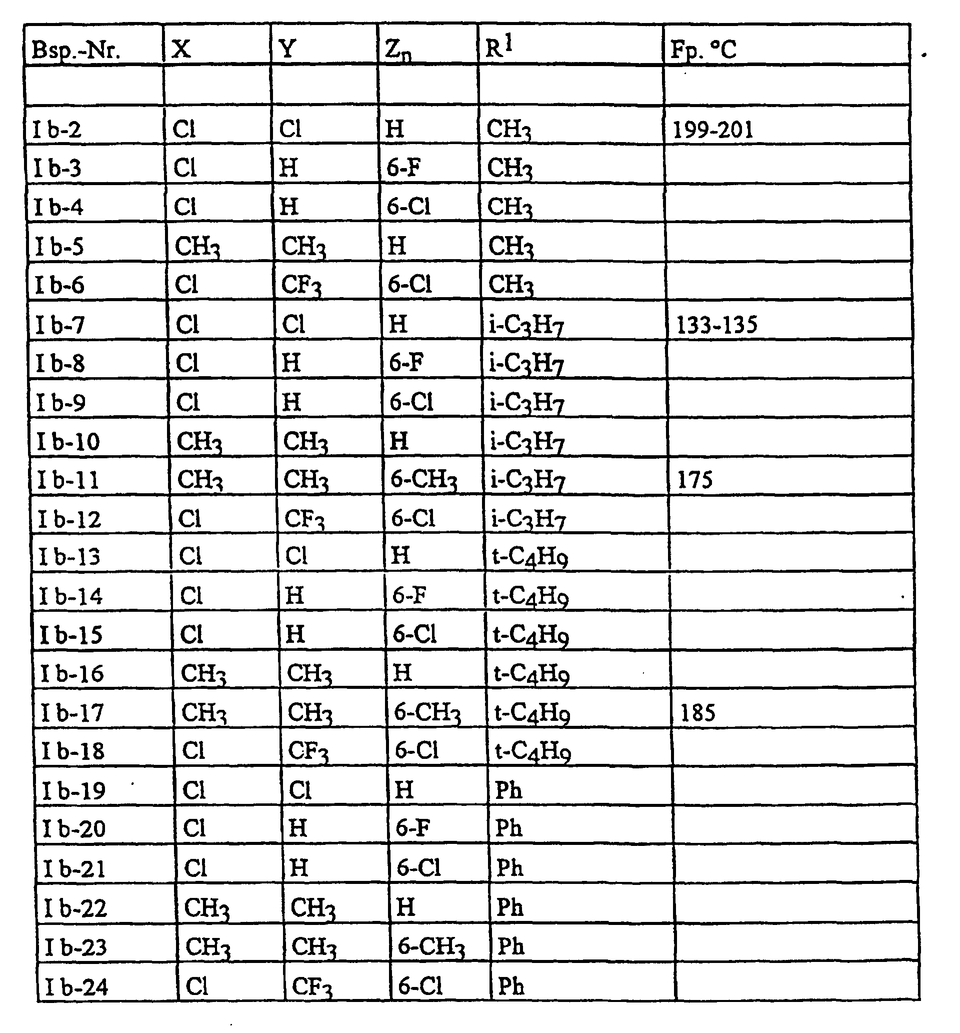

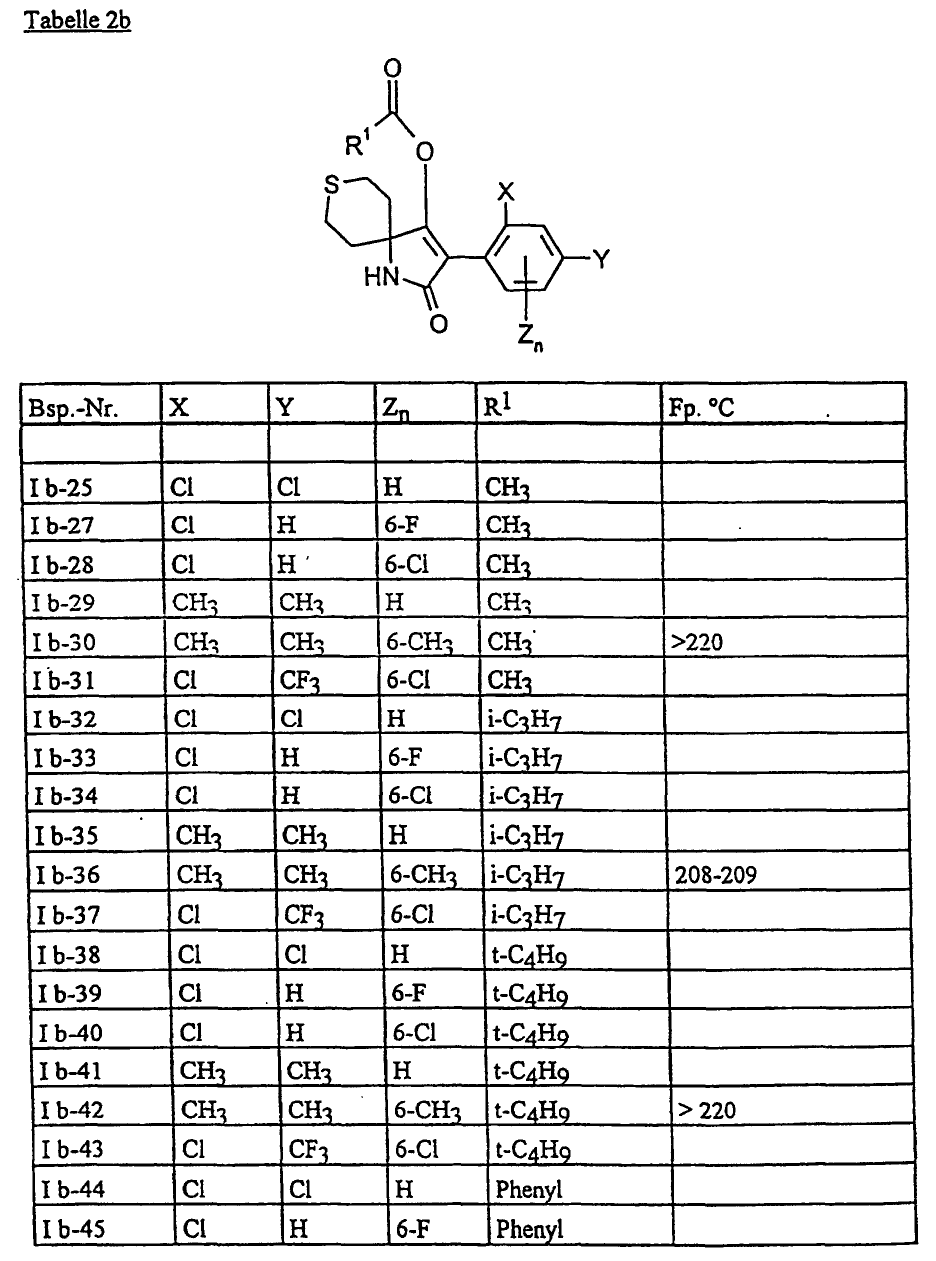

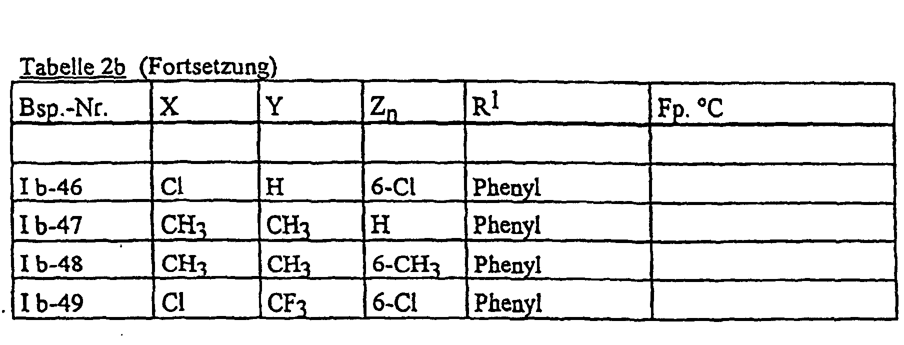

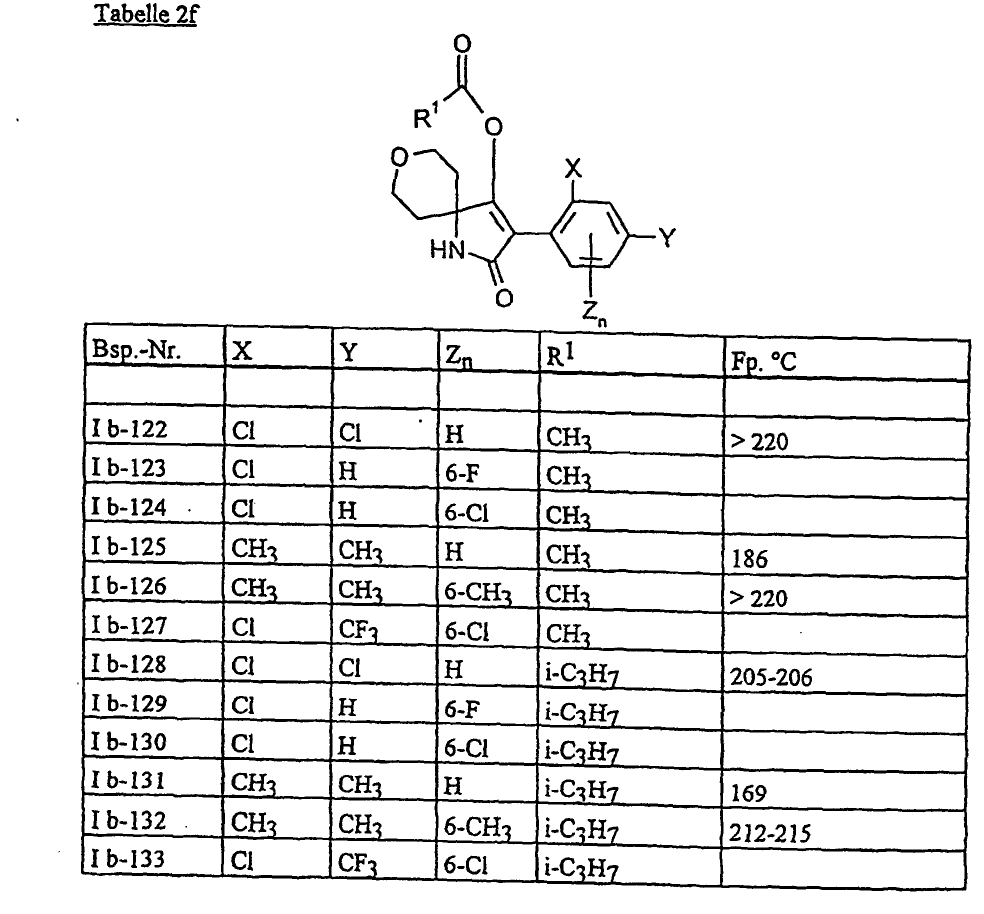

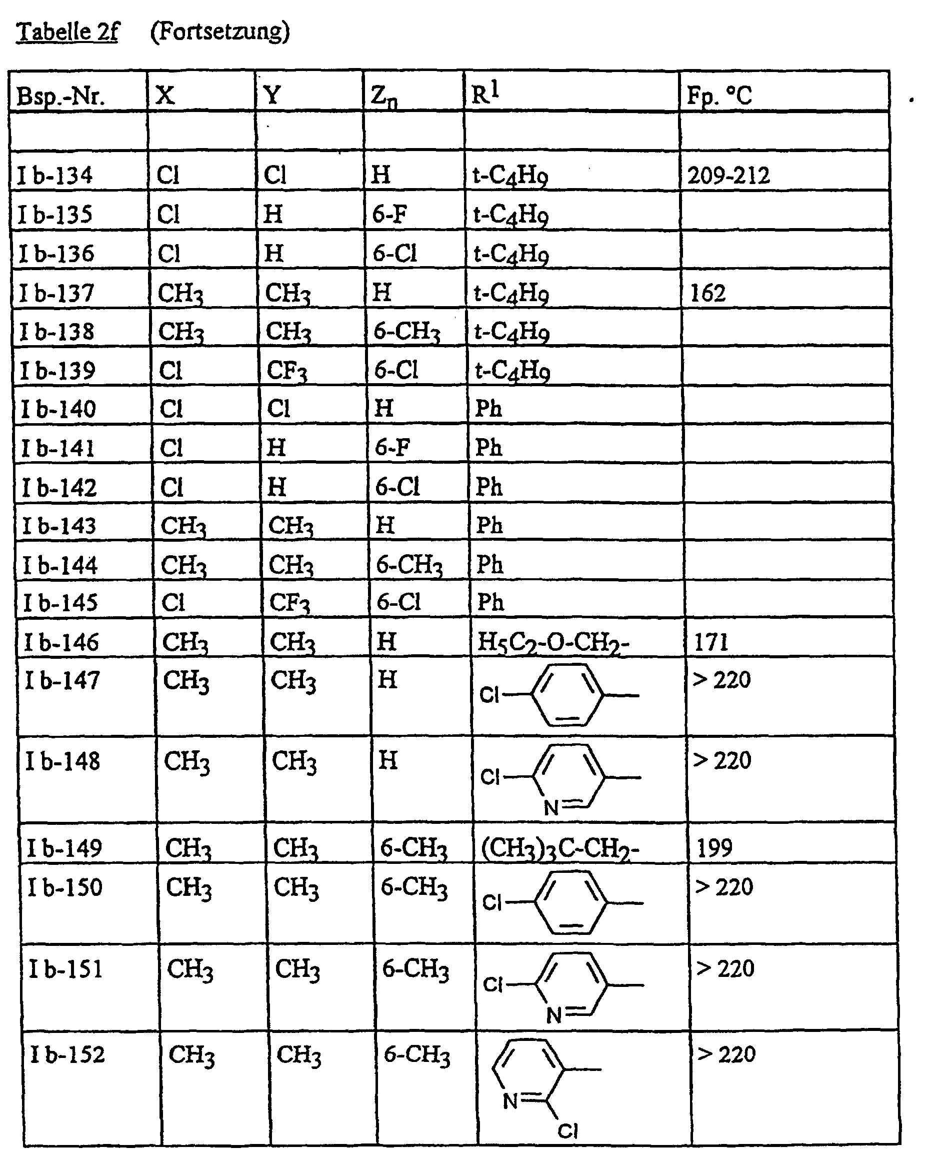

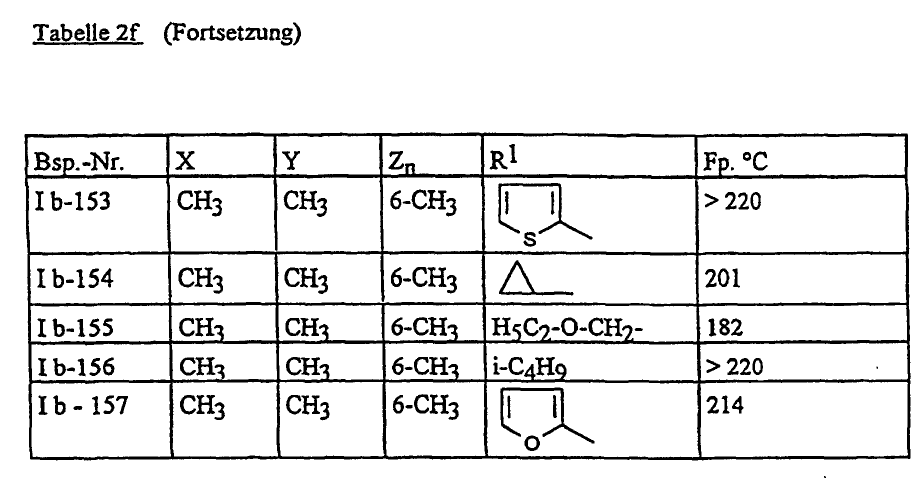

### Beispiel I c - 1

5,8 g der Verbindung des Beispiels I a-1 werden in 70 ml trockenem Methylenchlorid mit 2,8 ml Triethylamin versetzt und bei 0 bis 10°C 2,7 g Chlorameisensäure-sec.-butylester in 5 ml trockenem Methylenchlorid zugegeben. Die Reaktionslösung wird zweimal mit 200 ml 0,5 N Natronlauge gewaschen, über Magnesiumsulfat getrocknet und das Lösungsmittel abdestilliert. Es bleiben 1,7 g (22 % d. Th.) Fp. 157°C.

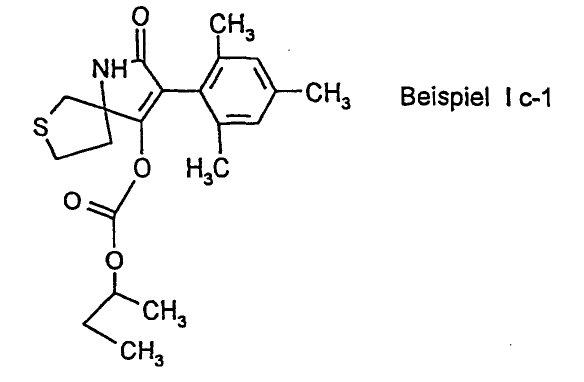

Analog werden die folgenden Verbindungen erhalten:

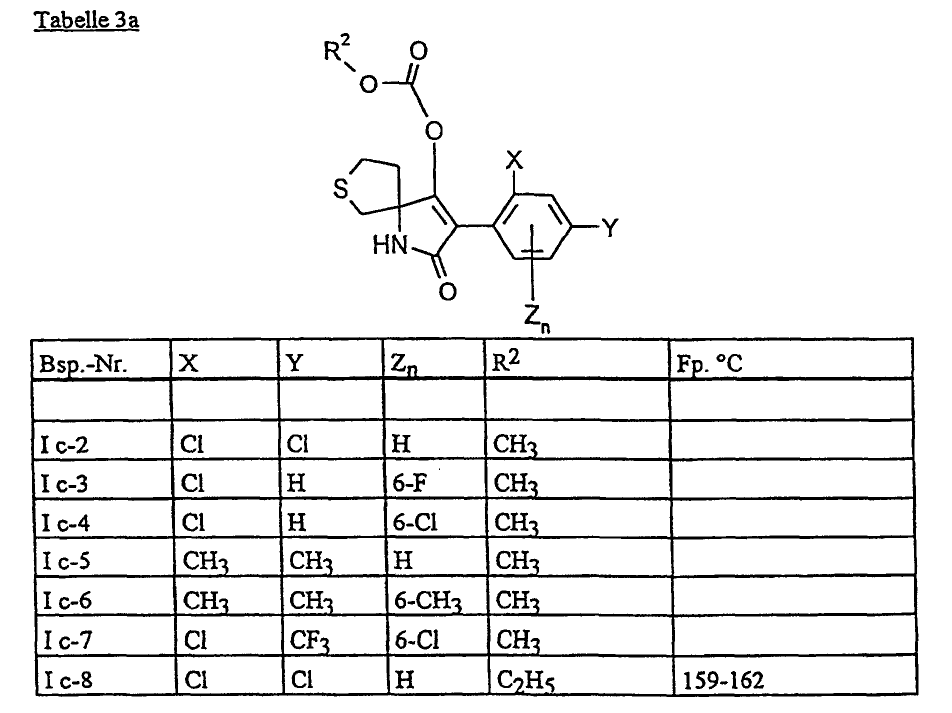

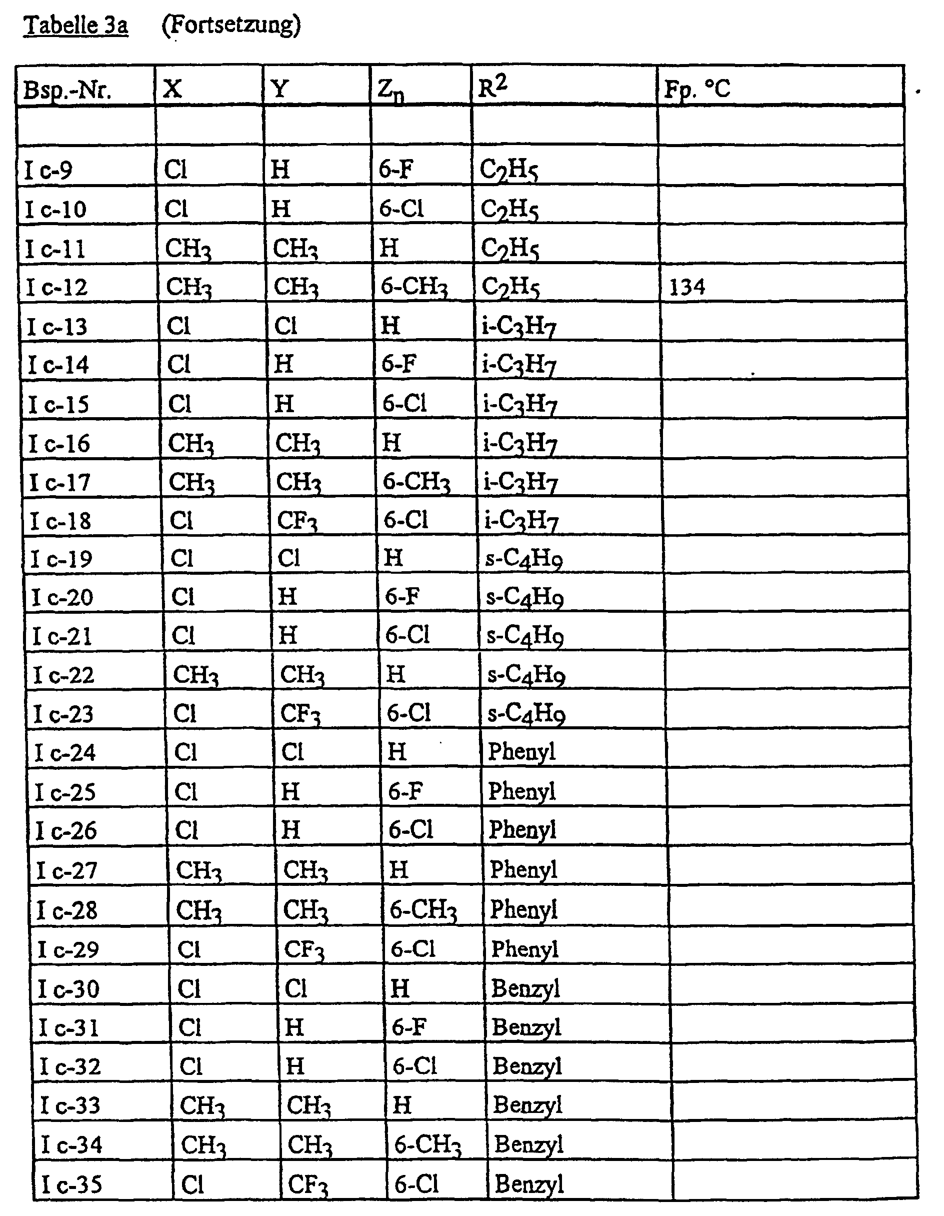

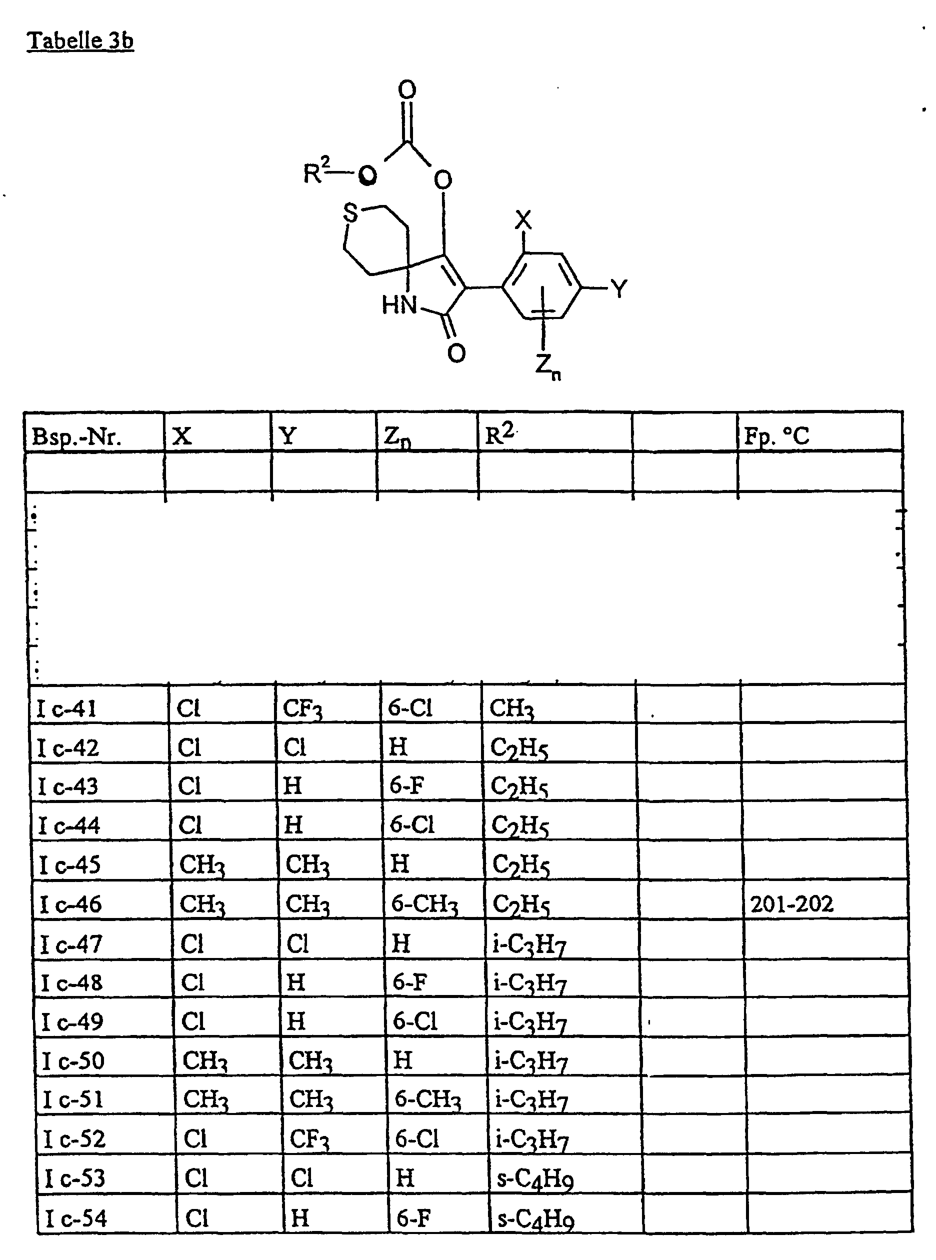

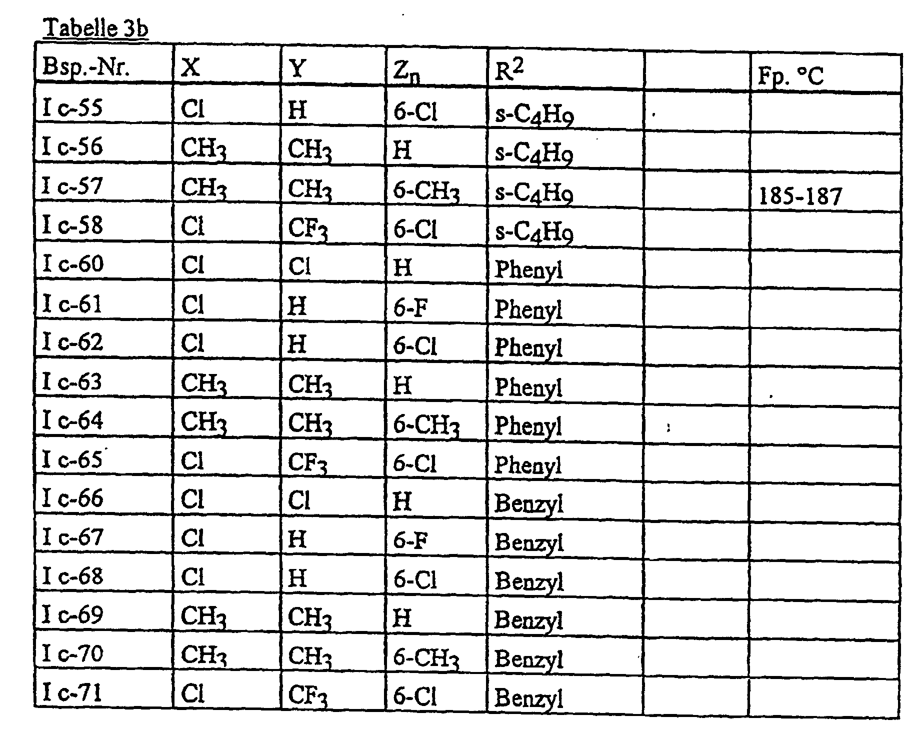

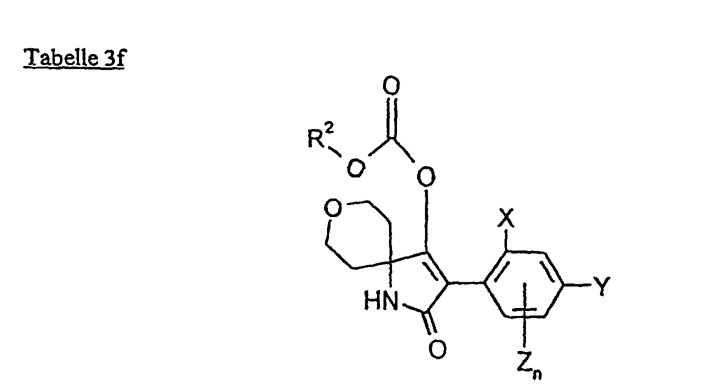

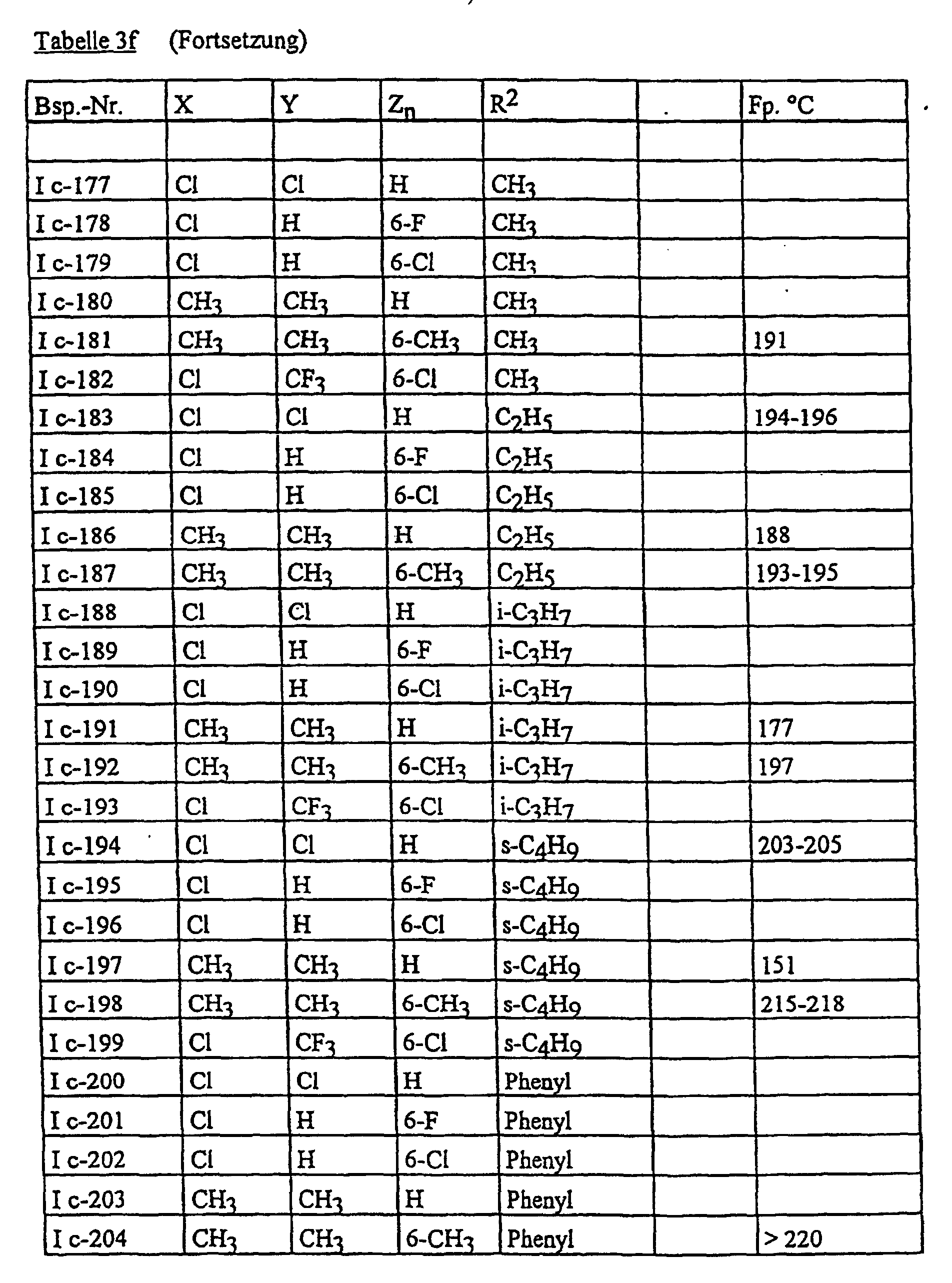

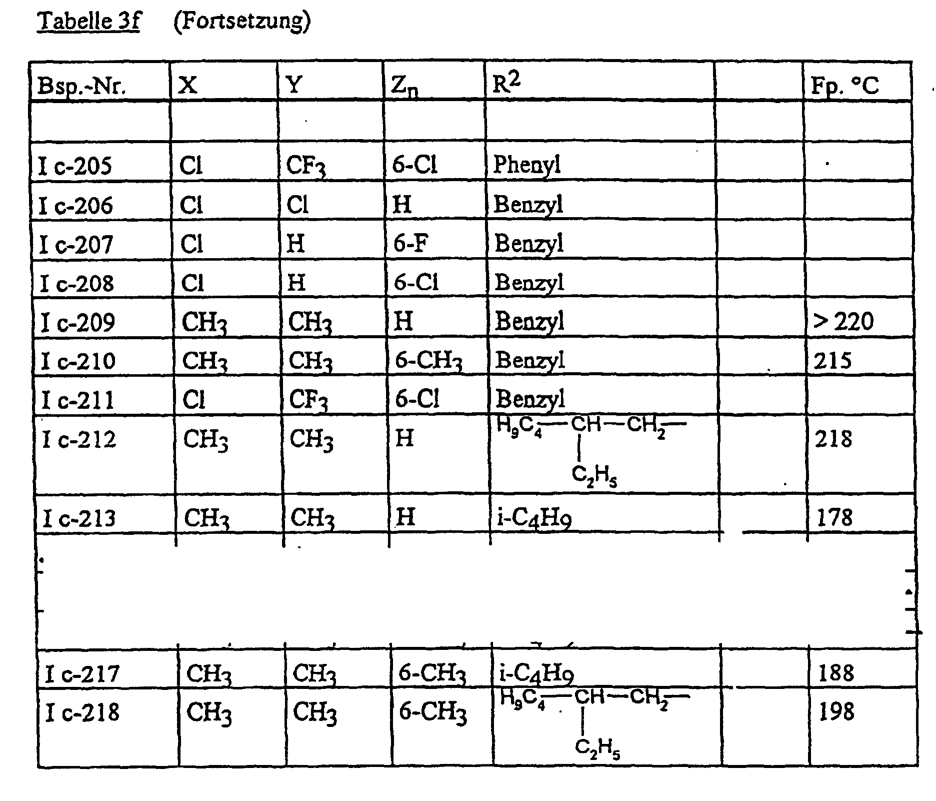

In den Anwendungsbeispielen wurden die folgenden aus dem Stand der Technik bekannten Vergleichsverbindungen eingesetzt: 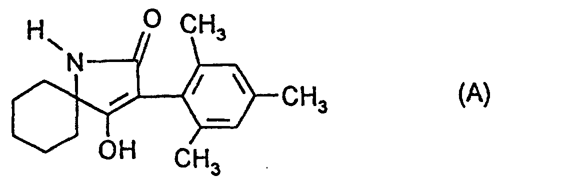 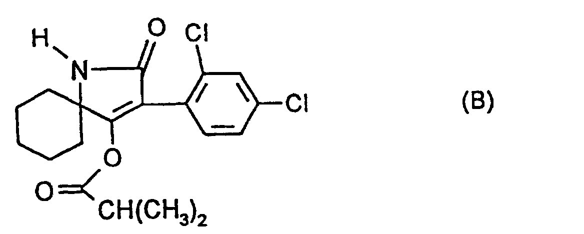 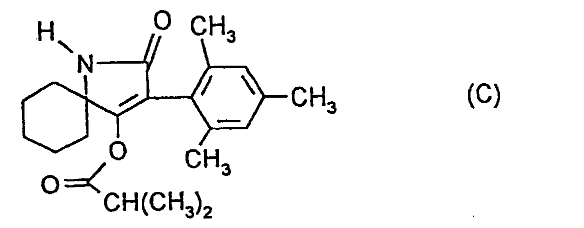 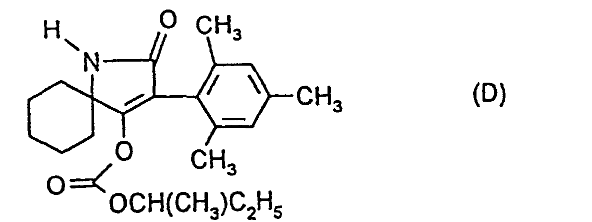 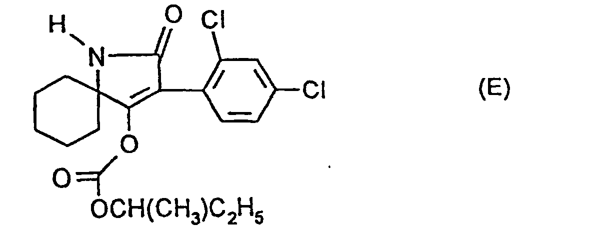 (alle bekannt aus EP 0456063)

### Beispiel A

| Myzus-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattlaus abgetötet wurde.

Bei diesem Test bewirkten z.B. die Verbindungen gemäß den Herstellungsbeispielen
Ib-128 bei einer beisgielhaften Wirkstoffkonzentration von 0,01 % eine Abtötung von mindestens 90% nach 6 Tagen, während die aus dem Stand der Technik bekannte Verbindung (A) keine Abtötung bewirkte und die aus dem Stand der Technik bekannte Verbindung (B) bei einer Wirkstoffkonzentration von 0,1% eine Abtötung von nur 20 % bewirkte.

### Beispiel B

| Plutella-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupe abgetötet wurde.

Bei diesem Test bewirkte z.B. die Verbindung gemäß Herstellungsbeispiel Ib-132 bei einer beispielhaften Wirkstoffkonzentration von 0,1 % eine Abtötung von 100 % nach 3 Tagen.

### Beispiel C

| Nephotettix - Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100%, daß alle Zikaden abgetötet wurden; 0 % bedeutet, daß keine Zikade abgetötet wurde.

Bei diesem Test bewirkten z.B. die Verbindungen gemäß den Herstellungsbeispielen Ic-194 und Ic-198 bei einer beispielhaften Wirkstoffkonzentration von 0,01 % eine Abtötung von 100 %.

### Beispiel D

| Tetranychus - Test (OP-resistent) | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe (Tetranychus urticae) befallen sind, werden mit einer Wirkstoffzubereitung der gewünschten Konzentration gespritzt.

Nach der gewünschten Zeit wird die Wirkung in % bestimmt. Dabei bedeutet 100%, daß alle Spinnmilben abgetötet wurden; 0 % bedeutet, daß keine Spinnmilbe abgetötet wurde.

Bei diesem Test bewirkten z.B. die Verbindungen gemäß den Herstellungsbeispielen
Ib-132 and Ic-198 bei einer beispielhaften Wirkstoffkonzentration von 0,02 % eine Abtötung von 100 % nach 14 Tagen.

### Beispiel E

| Panonychus - Test | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Ca. 30 cm hohe Pflaumenbäumchen (Prunus domestica), die stark von allen Entwicklungsstadien der Obstbaumspinnmilbe (Panonychus ulmi) befallen sind, werden mit einer Wirkstoffzubereitung der gewünschten Konzentration gespritzt.

Nach der gewünschten Zeit wird die Wirkung in % bestimmt. Dabei bedeutet 100%, daß alle Spinnmilben abgetötet wurden; 0 % bedeutet, daß keine Spinnmilbe abgetötet wurde.

Bei diesem Test bewirkten z.B. die Verbindungen gemäß den Herstellungsbeispielen
Ib-132 und Ic-198 bei einer beispielhaften Wirkstoffkonzentration von 0,02 % eine Abtötung von 100 % nach 14 Tagen.

## Patentansprüche

1. Substituierte spirocyclische 1-H-3-Aryl-pyrrolidin-2,4-dion-derivate der Folmel (I) 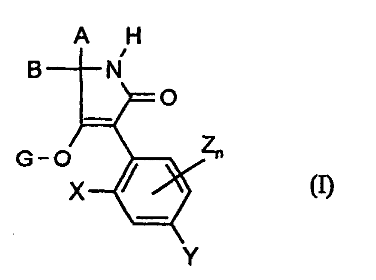 in welcher
A, B und das Kohlenstoffatom, an das sie gebunden sind, für einen 5- bis 6-gliedrigen Spirocyclus stehen, der Sauerstoff oder Schwefel enthält, der unsubstituiert ist,
X für C₁-C₆-Alkyl, Halogen oder C₁-C₆-Alkoxy steht,
Y für Wassertoff, C₁-C₆-Alkyl, Halogen, C₁-C₆-Alkoxy oder C₁-C₃-Halogenalkyl steht,
Z für C₁-C₆-Alkyl, Halogen oder C₁-C₆-Alkoxy steht,
G für die Gruppen 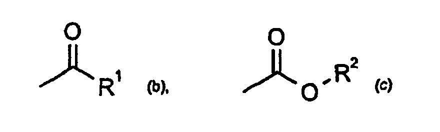 steht, in welchen
R¹ für jeweils gegebenenfalls durch Halogen substituiertes C₁-C₂₀-Alkyl, C₂-C₁₀-Alkenyl, C₁-C₈-Alkoxy-C₁-C₈-alkyl, C₁-C₈-Alkylthio-C₁-C₈-alkyl, C₁-C₈-Polyalkoxy-C₁-C₈-alkyl oder Cycloalkyl mit 3 bis 8 Ringatomen, das durch Sauerstoff- und/oder Schwefelatome unterbrochen und durch C₁-C₆-Alkyl substituiert sein kann,
für gegebenenfalls durch Halogen, Nitro, C₁-C₆-Alkyl, C₁-C₆-Alkoxy, C₁-C₆-Halogenalkyl oder C₁-C₆-Halogenalkoxy substituiertes Phenyl,
für gegebenenfalls durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl
für gegebenenfalls durch Halogen und/oder C₁-C₆-Alkyl substituiertes Hetaryl,
für gegebenenfalls durch Halogen und/oder C₁-C₆-Alkyl substituiertes Phenoxy-C₁-C₆-alkyl oder
für gegebenenfalls durch Halogen, Amino und/oder C₁-C₆-Alkyl substituiertes Hetaryloxy-C₁-C₆-alkyl steht,
R² für jeweils gegebenenfalls durch Halogen substituiertes C₁-C₂₀-Alkyl, C₃-C₁₀-Alkenyl, C₁-C₈-Alkoxy-C₂-C₈-alkyl, C₁-C₈-Polyalkoxy-C₂-C₈-alkyl
für gegebenenfalls durch Halogen oder C₁-C₆-Alkyl substituiertes C₃-C₆-Cycloalkyl oder
für jeweils gegebenenfalls durch Halogen, Nitro, C₁-C₆-Alkyl, C₁-C₆-Alkoxy oder C₁-C₆-Halogenalkyl substituiertes Phenyl oder Benzyl steht und
n für eine Zahl von 0 bis 3 steht.

2. Substituierte spirocyclische 1-H-3-Aryl-pyrrolidin-2,4-dionderivate gemäß Anspruch 1 der folgenden Formeln (Ib) bis (I-c): 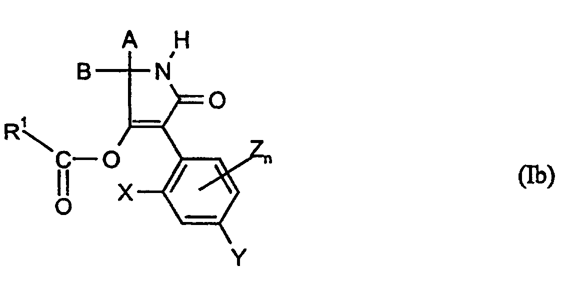 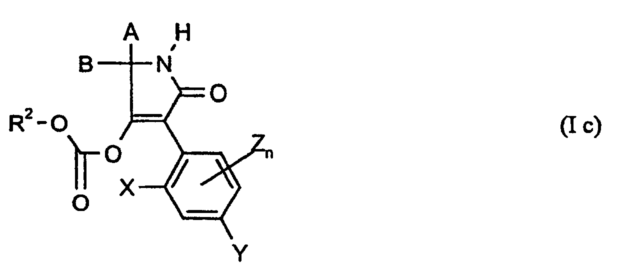 worin
A, B, X, Y, Z, R¹, R² und n
die in Anspruch 1 angegebenen Bedeutungen besitzen.

3. Substituierte spirocyclische 1H-3-Aryl-pyrrolidin-2,4-dionderivate der Formel (I) gemäß Anspruch 1, in welcher
A, B und das Koblenstoffatom, an das sie gebunden sind, für einen 5- bis 6-gliedrigen Spirocyclus stehen, der Sauerstoff oder Schwefel enthält, der unsubstituiert ist,
X für C₁-C₄-Alkyl, Halogen oder C₁-C₄-Alkoxy steht,
Y für Wasserstoff, C₁-C₄-Alkyl, Halogen, C₁-C₄-Alkoxy oder C₁-C₂-Halogenalkyl steht,
Z für C₁-C₄-Alkyl, Halogen oder C₁-C₄-Alkoxy steht,
G für die Gruppen 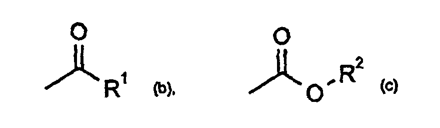 steht, in welchen
R¹ für jeweils gegebenenfalls durch Halogen substituiertes C₁-C₁₆-Alkyl, C₂-C₈-Alkenyl, C₁-C₆-Alkoxy-C₁-C₆-alkyl, C₁-C₆-Alkylthio-C₁-C₆-alkyl, C₁-C₆-Polyalkoxy-C₁-C₆-alkyl oder Cycloalkyl mit 3 bis 7 Ringatomen, das durch 1-2 Sauerstoff- und/oder Schwefelatome unterbrochen und durch C₁-C₄-Alkyl substituiert sein kann,
für gegebenenfalls durch Halogen, Nitro, C₁-C₄-Alkyl, C₁-C₄-Alkoxy, C₁-C₃-Halogenalkyl oder C₁-C₃-Halogenalkoxy substituiertes Phenyl,
für gegebenenfalls durch Halogen, C₁-C₄-Alkyl, C₁-C₄-Alk-oxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom und/oder C₁-C₄-Alkyl substituiertes Furanyl, Thienyl, Pyridyl, Pyrimidyl, Thiazolyl oder Pyrazolyl,
für gegebenenfalls durch Fluor, Chlor, Brom und/oder C₁-C₄-Alkyl substituiertes Phenoxy-C₁-C₅-alkyl oder
für jeweils gegebenenfalls durch Fluor, Chlor, Brom, Amino und/oder C₁-C₄-Alkyl substituiertes Pyridyloxy-C₁-C₆-alkyl, Pyrimidinyloxy-C₁-C₆-alkyl oder Thiazolyloxy-C₁-C₆-akyl steht,
R² für jeweils gegebenenfalls durch Halogen substituiertes C₁-C₁₆-Alkyl, C₃-C₈-Alkenyl, C₁-C₆-Alkoxy-C₂-C₆-alkyl, C₁-C₆-Polyalkoxy-C₂-C₆-alkyl,
für gegebenenfalls durch Fluor, Chlor oder C₁-C₄-Alkyl substituiertes C₃-C₆-Cycloalkyl oder
für jeweils gegebenenfalls durch Halogen, Nitro, C₁-C₄-Alkyl, C₁-C₃-Alkoxy oder C₁-C₃-Halogenalkyl substituiertes Phenyl oder Benzyl steht und
n für eine Zahl von 0 bis 2 steht.

4. Substituierte spirocyclische 1H-3-Aryl-pyrrolidin-2,4-dionderivate der Formel (I) gemäß Anspruch 1, in welcher
A, B und das Kohlenstoffatom, an das sie gebunden sind, für einen 5- bis 6-gliedrigen Spirocyclus stehen, der Sauerstoff oder Schwefel enthält, der unsubstituiert ist,
X für Methyl, Ethyl, Propyl, 2-Propyl, Fluor, Chlor, Brom, Methoxy oder Ethoxy steht,
Y für Wasserstoff, Methyl, Ethyl, Propyl, i-Propyl, Butyl, i-Butyl, tert.-Butyl, Fluor, Chlor, Brom, Methoxy, Ethoxy oder Trifluormethyl steht,
Z für Methyl, Ethyl, Propyl, i-Propyl, Butyl, i-Butyl, tert.-Butyl, Fluor, Chlor, Brom, Methoxy oder Ethoxy steht,
G für die Gruppen 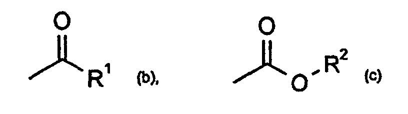 steht, in welchen
R¹ für jeweils gegebenenfalls durch Fluor oder Chlor substituiertes C₁-C₁₄-Alkyl, C₂-C₆-Alkenyl, C₁-C₄-Alkoxy-C₁-C₆-alkyl, C₁-C₄-Alkylthio-C₁-C₆-alkyl, C₁-C₄-Polyalkoxy-C₁-C₄-alkyl oder Cycloalkyl mit 3 bis 6 Ringatomen, das durch 1 bis 2 Sauerstoff- und/oder Schwefelatome unterbrochen und durch Methyl oder Ethyl substituiert sein kann,
für gegebenenfalls durch Fluor, Chlor, Brom, Methyl, Ethyl, Propyl, i-Propyl, Methoxy, Ethoxy, Trifluormethyl, Trifluormethoxy oder Nitro substituiertes Phenyl,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-allyl,
für jeweils gegebenenfalls durch Fluor, Chlor, Brom, Methyl, Ethyl substituiertes Furanyl, Thienyl, Pyridyl, Pyrimidyl, Thiazolyl oder Pyrazolyl,
für gegebenenfalls durch Fluor, Chlor, Methyl, Ethyl substituiertes Phenoxy-C₁-C₄-alkyl oder
für jeweils gegebenenfalls durch Fluor, Chlor, Amino, Methyl, Ethyl substituiertes Pyridyloxy-C₁-C₄-akyl, Pyrimidinyloxy-C₁-C₄-alkyl oder Thiazolyloxy-C₁-C₄-alkyl steht,
R² für jeweils gegebenenfalls durch Fluor oder Chlor substituiertes C₁-C₁₄-Alkyl, C₃-C₆-Alkenyl, C₁-C₄-Alkoxy-C₂-C₆-alkyl, C₁-C₄-Polyalkoxy-C₂-C₆-alkyl, für gegebenenfalls durch Fluor, Chlor, Methyl oder Ethyl substituiertes C₃-C₆-Cycloalkyl
oder für jeweils gegebenenfalls durch Fluor, Chlor, Nitro, Methyl, Ethyl, Propyl, i-Propyl, Methoxy, Ethoxy, Trifluormethyl substituiertes Phenyl oder Benzyl steht und
n für 0 oder 1 steht.

5. Verfahren zur Herstellung der spirocyclischen substituierten 1H-3-Arylpyrrolidin-2,4-dionderivate der Formel (I) gemäß Anspruch 1
**dadurch gekennzeichnet, daß** man
(B) daß man Verbindungen der Formel (Ib) 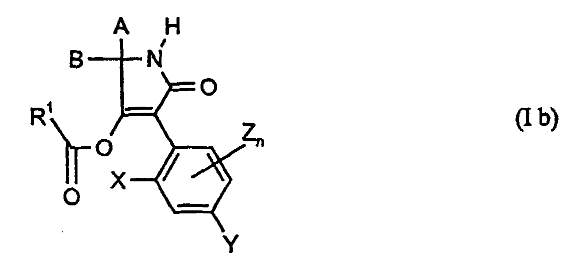 in welcher
A, B, X, Y, Z, R¹ und n die oben angegebene Bedeutung haben, erhält, wenn man
Verbindungen der Formel (Ia), 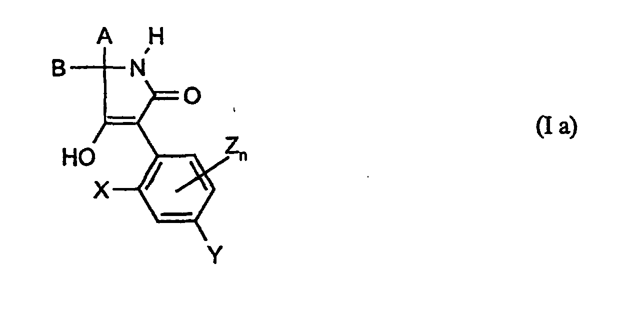 in welcher
A, B, X, Y, Z und n die oben angegebene Bedeutung haben,
α) mit Säurehalogeniden der Formel (III) 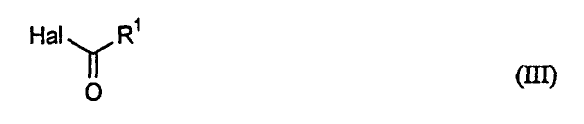 in welcher
R¹ die oben angegebene Bedeutung hat und
Hal für Halogen steht,
gegebenenfalls in Gegenwart eines Verdünnungsmittels und gegebenenfalls in Gegenwart eines Säurebindemittels umsetzt
oder
β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels, umsetzt;
oder
(C) daß man Verbindungen der Formel (Ic-1) 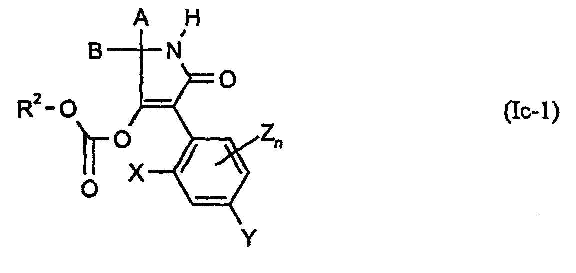 in welcher
A, B, X, Y, Z, R² und n die oben angegebene Bedeutung haben, erhält, wenn man
Verbindungen der Formel (Ia) 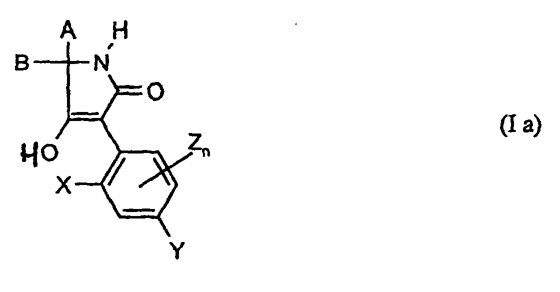 in welcher
A, B, X, Y, Z und n die oben angegebene Bedeutung haben,
mit Chlorameisensäureester der Formel (V)
R²-O-CO-Cl (V)
in welcher
R² die oben angegebene Bedeutung hat,
gegebenenfalls in Gegenwart eines Verdünnungsmittels und gegebenenfalls in Gegenwart eines Säurebindemittels umsetzt.

6. Schädlingsbekämpfungsmittel und Herbizide, **gekennzeichnet durch** einen Gehalt an mindestens einem substituierten spirocyclischen 1-H-3-Arylpyrrolidin-2,4-dion-derivat der Formel (I) gemäß Anspruch 1.

7. Verwendung von substituierte spirocyclischen 1-H-3-Aryl-pyrrolidin-2,4-dion-derivaten der Formel (I) gemäß Anspruch 1 zur Bekämpfung von Schädlingen und unerwünschtem Pflanzenbewuchs.

8. Verfahren zur Bekämpfung von Schädlingen, **dadurch gekennzeichnet, daß** man substituierte spirocyclische 1-H-3-Aryl-pyrrolidin-2,4-dion-derivate der Formel (I) gemäß Anspruch 1 auf Schädlinge, unerwünschten Pflanzenbewuchs und/oder ihren Lebensraum einwirken läßt.

9. Verfahren zur Herstellung von Schädlingsbekämpfungsmitteln und Herbiziden, **dadurch gekennzeichnet, daß** man substituierte spirocyclische 1-H-3-Aryl-pyrrolidin-2,4-dion-derivate der Formel (I) gemäß Anspruch 1 mit Streckmitteln und/oder oberflächenaktiven Mitteln vermischt.

## Claims

1. Substituted spirocyclic 1-H-3-aryl-pyrrolidine-2,4-dione derivatives of the formula (I) 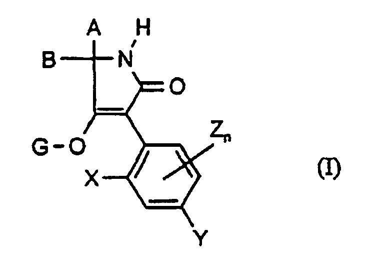 in which
A, B and the carbon atom to which they are bonded represent a 5- to 6-membered spirocycle which contains oxygen or sulphur and which is unsubstituted,
X represents C₁-C₆-alkyl, halogen or C₁-C₆-alkoxy,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
G represents the groups 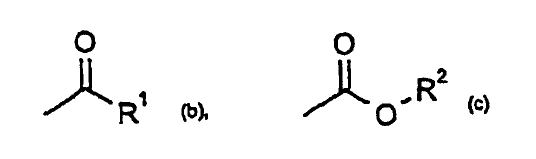 in which
R¹ represents in each case optionally halogen-substituted C₁-C₂₀-alkyl, C₂-C₁₀-alkenyl, C₁-C₈-alkoxy-C₁-C₈-alkyl, C₁-C₈-alkylthio-C₁-C₈-alkyl, C₁-C₈-polyalkoxy-C₁-C₈-alkyl or cycloalkyl which has 3 to 8 ring atoms and which can be interrupted by oxygen and/or sulphur atoms and substituted by C₁-C₆-alkyl,
phenyl which is optionally substituted by halogen, nitro, C₁-C₆-alkyl, C₁-C₆-alkoxy, C₁-C₆-halogenoalkyl or C₁-C₆-halogenoalkoxy,
phenyl-C₁-C₆-alkyl which is optionally substituted by halogen, C₁-C₆-alkyl, C₁-C₆-alkoxy, C₁-C₆-halogenoalkyl or C₁-C₆-halogenoalkoxy
hetaryl which is optionally substituted by halogen and/or C₁-C₆-alkyl,
phenoxy-C₁-C₆-alkyl which is optionally substituted by halogen and/or C₁-C₆-alkyl or
hetaryloxy-C₁-C₆-alkyl which is optionally substituted by halogen, amino and/or C₁-C₆-alkyl,
R² represents C₁-C₂₀-alkyl, C₃-C₁₀-alkenyl, C₁-C₈-alkoxy-C₂-C₈-alkyl, C₁-C₈-polyalkoxy-C₂-C₈-alkyl, each of which is optionally substituted by halogen,
C₃-C₆-cycloalkyl which is optionally substituted by halogen or C₁-C₆-alkyl or
phenyl or benzyl, each of which is optionally substituted by halogen, nitro, C₁-C₆-alkyl, C₁-C₆-alkoxy or C₁-C₆-halogenoalkyl and
n represents an integer from 0 to 3.

2. Substituted spirocyclic 1-H-3-aryl-pyrrolidine-2,4-dione derivatives according to Claim 1 of the following formulae (Ib) to (Ic): 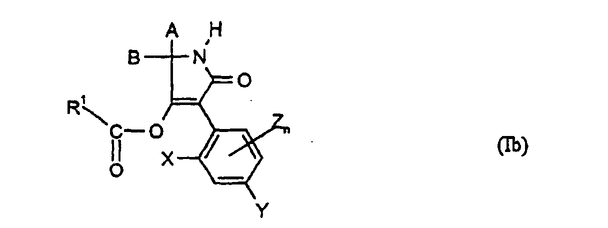 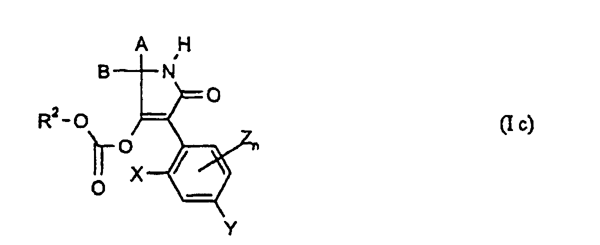 in which
A, B, X, Y, Z, R¹, R² and n
have the meanings given in Claim 1.

3. Substituted spirocyclic 1H-3-aryl-pyrrolidine-2,4-dione derivatives of the formula (I) according to Claim 1 in which
A, B and the carbon atom to which they are bonded represent a 5- to 6-membered spirocycle which contains oxygen or sulphur and which is unsubstituted
X represents C₁-C₄-alkyl, halogen or C₁-C₄-alkoxy,
Y represents hydrogen, C₁-C₄-alkyl, halogen, C₁-C₄-alkoxy or C₁-C₂-halogenoalkyl,
Z represents C₁-C₄-alkyl, halogen or C₁-C₄-alkoxy,
G represents the groups 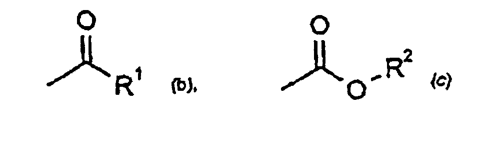 in which
R¹ represents in each case optionally halogen-substituted C₁-C₁₆-alkyl, C₂-C₈-alkenyl, C₁-C₆-alkoxy-C₁-C₆-alkyl, C₁-C₆-alkylthio-C₁-C₆-alkyl, C₁-C₆-polyalkoxy-C₁-C₆-alkyl or cycloalkyl which has 3 to 7 ring atoms and which can be interrupted by 1-2 oxygen and/or sulphur atoms and substituted by C₁-C₄-alkyl,
phenyl which is optionally substituted by halogen, nitro, C₁-C₄-alkyl, C₁-C₄-alkoxy, C₁-C₃-halogenoalkyl or C₁-C₃-halogenoalkoxy,
phenyl-C₁-C₄-alkyl which is optionally substituted by halogen, C₁-C₄-alkyl, C₁-C₄-alkoxy, C₁-C₃-halogenoalkyl or C₁-C₃-halogenoalkoxy,
furanyl, thienyl, pyridyl, pyrimidyl, thiazolyl or pyrazolyl, each of which is optionally substituted by fluorine, chlorine, bromine and/or C₁-C₄-alkyl,
phenoxy-C₁-C₅-alkyl which is optionally substituted by fluorine, chlorine, bromine and/or C₁-C₄-alkyl or
pyridyloxy-C₁-C₆-alkyl, pyrimidinyloxy-C₁-C₆-alkyl or thiazolyloxy-C₁-C₆-alkyl, each of which is optionally substituted by fluorine, chlorine, bromine, amino and/or C₁-C₄-alkyl,
R² represents C₁-C₁₆-alkyl, C₃-C₈-alkenyl, C₁-C₆-alkoxy-C₂-C₆-alkyl, C₁-C₆-polyalkoxy-C₂-C₆-alkyl, each of which is optionally substituted by halogen,
C₃-C₆-cycloalkyl which is optionally substituted by fluorine, chlorine or C₁-C₄-alkyl or
phenyl or benzyl which is optionally substituted by halogen, nitro, C₁-C₄-alkyl, C₁-C₃-alkoxy or C₁-C₃-halogenoalkyl and
n represents an integer from 0 to 2.

4. Substituted spirocyclic lH-3-aryl-pyrrolidine-2,4-dione derivatives of formula (I) according to Claim 1 in which
A, B and the carbon atom to which they are bonded represent a 5- to 6-membered spirocycle which contains oxygen or sulphur and which is unsubstituted
X represents methyl, ethyl, propyl, 2-propyl, fluorine, chlorine, bromine, methoxy or ethoxy,
Y represents hydrogen, methyl, ethyl, propyl, i-propyl, butyl, i-butyl, tert-butyl, fluorine, chlorine, bromine, methoxy, ethoxy or trifluoromethyl,
Z represents methyl, ethyl, propyl, i-propyl, butyl, i-butyl, tert-butyl, fluorine, chlorine, bromine, methoxy or ethoxy,
G represents the groups 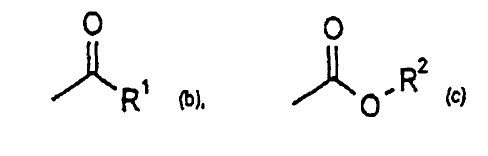 in which
R¹ represents in each case fluorine- or chlorine-substituted C₁-C₁₄-alkyl, C₂-C₆-alkenyl, C₁-C₄-alkoxy-C₁-C₆-alkyl, C₁-C₄-alkylthio-C₁-C₆-alkyl, C₁-C₄-polyalkoxy-C₁-C₄-alkyl or cycloalkyl which has 3 to 6 ring atoms and which can be interrupted by 1 to 2 oxygen and/or sulphur atoms and substituted by methyl or ethyl,
phenyl which is optionally substituted by fluorine, chlorine, bromine, methyl, ethyl, propyl, i-propyl, methoxy, ethoxy, trifluoromethyl, trifluoromethoxy or nitro,
phenyl-C₁-C₃-alkyl which is optionally substituted by fluorine, chlorine, bromine, methyl, ethyl, propyl, i-propyl, methoxy, ethoxy, trfluoromethyl, trifluoromethoxy,
furanyl, thienyl, pyridyl, pyrimidyl, thiazolyl or pyrazolyl, each of which is optionally substituted by fluorine, chlorine, bromine, methyl, ethyl,
phenoxy-C₁-C₄-alkyl which is optionally substituted by fluorine, chlorine, methyl, ethyl, or
pyridyloxy-C₁-C₄-alkyl, pyrimidinyloxy-C₁-C₄-alkyl or thiazolyloxy-C₁-C₄-alkyl, each of which is optionally substituted by fluorine, chlorine, amino, methyl, ethyl,
R² represents C₁-C₁₄-alkyl, C₃-C₆-alkenyl, C₁-C₄-alkoxy-C₂-C₆-alkyl, C₁-C₄-polyalkoxy-C₂-C₆-alkyl, each of which is optionally substituted by fluorine or chlorine, or represents C₃-C₆-cycloalkyl which is optionally substituted by fluorine, chlorine, methyl or ethyl,
or represents phenyl or benzyl, each of which is optionally substituted by fluorine, chlorine, nitro, methyl, ethyl, propyl, i-propyl, methoxy, ethoxy, trifluoromethyl, and
n represents 0 or 1.

5. Process for the preparation of the spirocyclic substituted 1H-3-aryl-pyrrolidine-2,4-dione derivatives of the formula (I) according to Claim 1,
**characterized in that**
(B) compounds of the formula (Ib) 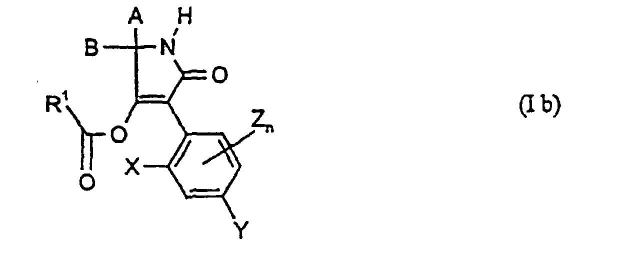 in which
A, B, X, Y, Z, R¹ and n are as stated above ar obtained when
compounds of the formula (Ia) 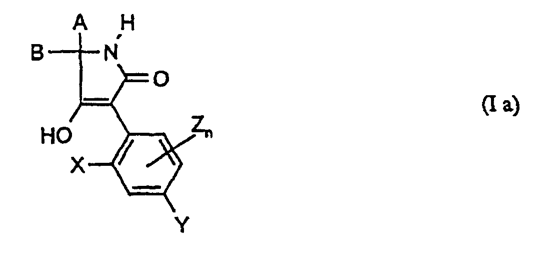 in which
A, B, X, Y, Z and n are as stated above
are reacted
α) with acid halides of the formula (III) 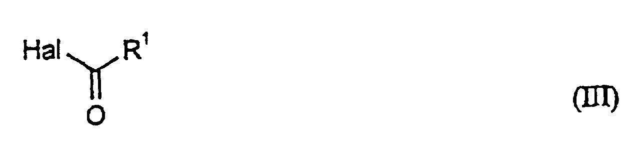 in which
R¹ is as stated above and
Hal represents halogen,
if appropriate in the presence of a diluent and if appropriate in the presence of an acid-binding agent
or
β) with carboxylic anhydrides of the formula (IV)
R¹-CO-O-CO-R¹ (IV)
in which
R¹ is as stated above,
if appropriate in the presence of a diluent and if appropriate in the presence of an acid-binding agent;
or
(C) compounds of the formula (Ic-1) 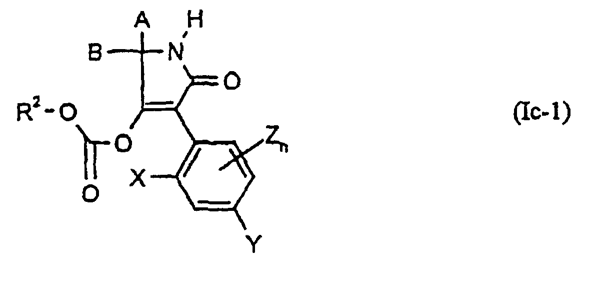 in which
A, B, X, Y, Z, R² and n are as stated above are obtained when
compounds of the formula (Ia) 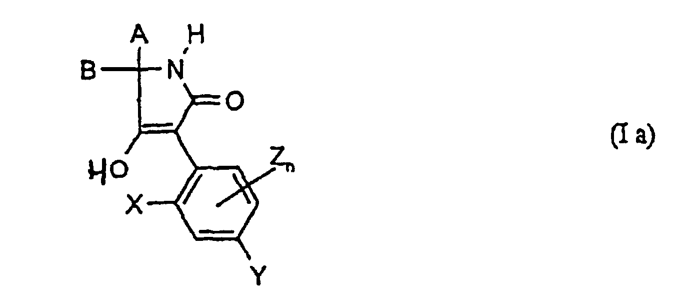 in which
A, B, X, Y, Z and n are as stated above are reacted with chloroformic ester of the formula (V)
R²-O-CO-Cl (V)
in which
R² is as stated above,
if appropriate in the presence of a diluent and if appropriate in the presence of an acid-binding agent.

6. Pesticides and herbicides, **characterized by** a content of at least one substituted spirocyclic 1-H-3-aryl-pyrrolidine-2,4-dione derivative of the formula (I) according to Claim 1.

7. Use of substituted spirocyclic 1-H-3-aryl-pyrrolidine-2,4-dione derivatives of the formula (I) according to Claim 1 for controlling pests and undesired vegetation.

8. Method of controlling pests, **characterized in that** substituted spirocyclic 1-H-3-aryl-pyrrolidine-2,4-dione derivatives of formula (I) according to Claim 1 are allowed to act on pests, undesired vegetation and/or their environment.

9. Process for the preparation of pesticides and herbicides, **characterized in that** substituted spirocyclic 1-H-3-aryl-pyrrolidine-2,4-dione derivatives of the formula (I) according to Claim 1 are mixed with extenders and/or surface-active agents.

## Revendications

1. Dérivés spirocycliques substitués de 1-H-3-aryl-pyrrolidine-2,4-diones, de formule (I) 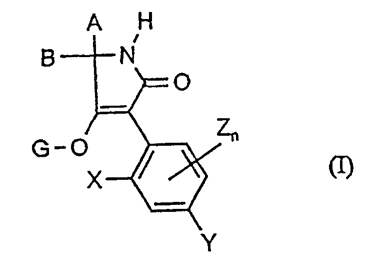 dans laquelle
A, B et l'atome de carbone auquel ils sont liés représentent un cycle spiranique pentagonal ou hexagonal contenant de l'oxygène ou du soufre, qui n'est pas substitué,
X est un groupe alkyle en C₁ à C₆, un halogène ou un groupe alkoxy en C₁ à C₆,
Y représente l'hydrogène, un groupe alkyle en C₁ à C₆, un halogène, un groupe alkoxy en C₁ à C₆ ou un groupe halogénalkyle en C₁ à C₃,
Z représente un groupe alkyle en C₁ à C₆, un halogène ou un groupe alkoxy en C₁ à C₆,
G représente les groupes 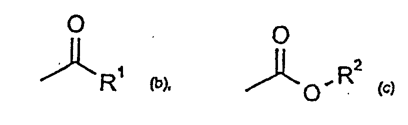 dans lesquels
R¹ représente un groupe, éventuellement substitué dans chaque cas par un halogène, alkyle en C₁ à C₂₀, alcényle en C₂ à C₁₀, (alkoxy en c₁ à C₈)-(alkyle en C₁ à C₈), (alkylthio en C₁ à C₈)-(alkyle en C₁ à C₈), poly(alkoxy en C₁ à C₈)-(alkyle en C₁ à C₈) ou cycloalkyle à noyau de 3 à 8 atomes qui peut être interrompu par des atomes d'oxygène et/ou de soufre et substitué par un radical alkyle en C₁ à C₆,
un groupe phényle portant éventuellement un substituant halogéno, nitro, alkyle en C₁ à C₆, alkoxy en C₁ à C₆, halogénalkyle en C₁ à C₆ ou halogénalkoxy en C₁ à C₆,
un groupe phényl-(alkyle en C₁ à C₆) portant éventuellement un substituant halogéno, alkyle en C₁ à C₆, alkoxy en C₁ à C₆, halogénalkyle en C₁ à C₆ ou halogénalkoxy en C₁ à C₆,
un groupe hétaryle portant éventuellement un substituant halogéno et/ou alkyle en C₁ à C₆,
un groupe phénoxy-(alkyle en C₁ à C₆) portant éventuellement un substituant halogéno et/ou alkyle en C₁ à C₆, ou bien
un groupe hétaryloxy-(alkyle en C₁ à C₆) portant éventuellement un substituant halogéno, amino et/ou alkyle en C₁ à C₆,
R² représente un groupe, éventuellement substitué dans chaque cas par un halogène, alkyle en C₁ à C₂₀, alcényle en C₃ à C₁₀, (alkoxy en C₁ à C₈)-(alkyle en C₂ à C₈), poly(alkoxy en C₁ à C₈)-(alkyle en C₂ à C₈),
un groupe cycloalkyle en C₃ à C₆ portant éventuellement un substituant halogéno ou alkyle en C₁ à C₆, ou bien
un groupe phényle ou un groupe benzyle dont chacun porte éventuellement un substituant halogéno, nitro, alkyle en C₁ à C₆, alkoxy en C₁ à C₆ ou halogénalkyle en C₁ à C₆, et
n représente un nombre de 0 à 3.

2. Dérivés spirocycliques substitués de 1-H-3-aryl-pyrrolidine-2,4-diones suivant la revendication 1, de formules générales (Ib) à (Ic) : 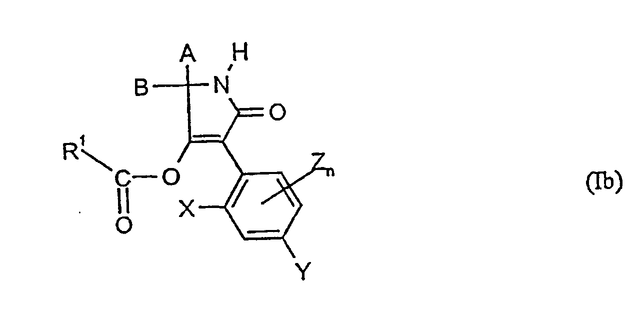 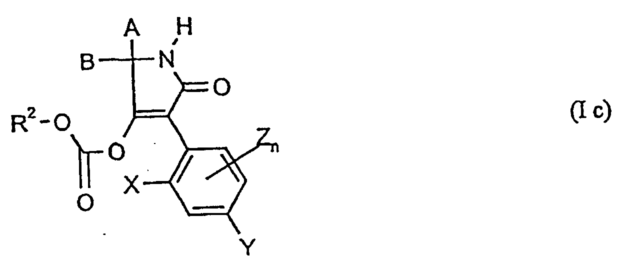 où
A, B, X, Y, Z, R¹, R² et n possèdent les définitions indiquées dans la revendication 1.

3. Dérivés spirocycliques substitués de 1H-3-aryl-pyrrolidine-2,4-diones de formule (I) suivant la revendication 1, dans laquelle
A, B et l'atome de carbone auquel ils sont liés représentent un cycle spiranique pentagonal ou hexagonal contenant de l'oxygène ou du soufre, qui n'est pas substitué,
X représente un groupe alkyle en C₁ à C₄, un halogène ou un groupe alkoxy en C₁ à C₄,
Y représente l'hydrogène, un groupe alkyle en C₁ à C₄, un halogène, un groupe alkoxy en C₁ à C₄ ou un groupe halogénalkyle en C₁ ou C₂,
Z représente un groupe alkyle en C₁ à C₄, un halogène ou un groupe alkoxy en C₁ à C₄,
G représente les groupes 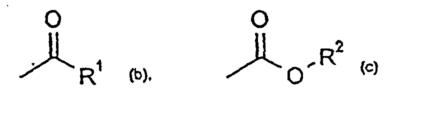 dans lesquels
R¹ représente un groupe, éventuellement substitué dans chaque cas par un halogène, alkyle en C₁ à c₁₆, alcényle en C₂ à C₈, (alkoxy en C₁ à C₆)-(alkyle en C₁ à C₆), (alkylthio en C₁ à C₆)-(alkyle en C₁ à C₆), poly(alkoxy en C₁ à C₆)-(alkyle en C₁ à C₆) ou cyclo-alkyle à noyau de 3 à 7 atomes qui peut être interrompu par 1-2 atomes d'oxygène et/ou de soufre et substitué par un radical alkyle en C₁ à C₄,
un groupe phényle portant éventuellement un substituant halogéno, nitro, alkyle en C₁ à C₄, alkoxy en C₁ à C₄, halogénalkyle en C₁ à C₃ ou halogénalkoxy en C₁ à C₃,
un groupe phényl-(alkyle en C₁ à C₄) portant éventuellement un substituant halogéno, alkyle en C₁ à C₄, alkoxy en C₁ à C₄, halogénalkyle en C₁ à C₃ ou halogénalkoxy en C₁ à C₃,
un groupe furannyle, thiényle, pyridyle, pyrimidyle, thiazolyle ou pyrazolyle, chacun étant éventuellement substitué par du fluor, du chlore, du brome et/ou un radical alkyle en C₁ à C₄,
un groupe phénoxy-(alkyle en C₁ à C₅) portant éventuellement un substituant fluoro, chloro, bromo et/ou alkyle en C₁ à C₄, ou bien
un groupe, éventuellement substitué dans chaque cas par un radical fluoro, chloro, bromo, amino et/ou alkyle en C₁ à C₄, pyridyloxy-(alkyle en C₁ à C₆), pyrimidinyloxy-(alkyle en C₁ à C₆) ou thiazolyloxy-(alkyle en C₁ à C₆),
R² est un groupe, éventuellement substitué dans chaque cas par un halogène, alkyle en C₁ à C₁₆, alcényle en C₃ à C₈, (alkoxy en C₁ à C₆)-(alkyle en C₂ à C₆), poly(alkoxy en C₁ à C₆)-(alkyle en C₂ à C₆),
un groupe cycloalkyle en C₃ à C₆ portant éventuellement un substituant fluoro, chloro ou alkyle en C₁ à C₄, ou bien
un groupe phényle ou un groupe benzyle dont chacun porte éventuellement un substituant halogéno, nitro, alkyle en C₁ à C₄, alkoxy en C₁ à C₃ ou halogénalkyle en C₁ à C₃, et
n représente un nombre de 0 à 2.

4. Dérivés spirocycliques substitués de 1H-3-aryl-pyrrolidine-2,4-diones de formule (I) suivant la revendication 1, dans laquelle
A, B et l'atome de carbone auquel ils sont liés représentent un cycle spiranique pentagonal ou hexagonal contenant de l'oxygène ou du soufre, qui n'est pas substitué,
X est un groupe méthyle, éthyle, propyle, 2-propyle, du fluor, du chlore, du brome, un groupe méthoxy ou éthoxy,
Y représente l'hydrogène, un groupe méthyle, éthyle, propyle, isopropyle, butyle, isobutyle, tertio-butyle, du fluor, du chlore, du brome, un groupe méthoxy, éthoxy ou trifluorométhyle,
Z représente un groupe méthyle, éthyle, propyle, isopropyle, butyle, isobutyle, tertio-butyle, du fluor, du chlore, du brome, un groupe méthoxy ou éthoxy,
G représente les groupes 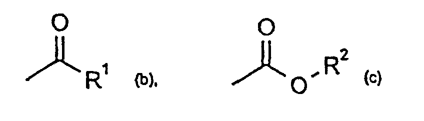 dans lesquels
R¹ est un groupe, éventuellement substitué dans chaque cas par du fluor ou du chlore, alkyle en C₁ à C₁₄, alcényle en C₂ à C₆, (alkoxy en C₁ à C₄)-(alkyle en C₁ à C₆), (alkylthio en C₁ à C₄)-(alkyle en C₁ à C₆), poly(alkoxy en C₁ à C₄)-(alkyle en C₁ à C₄) ou cyclo-alkyle à noyau de 3 à 6 atomes qui peut être interrompu par 1 à 2 atomes d'oxygène et/ou de soufre et substitué par un radical méthyle ou éthyle,
un groupe phényle portant éventuellement un substituant fluoro, chloro, bromo, méthyle, éthyle, propyle, isopropyle, méthoxy, éthoxy, trifluorométhyle, trifluorométhoxy ou nitro,
un groupe phényl-(alkyle en C₁ à C₃) portant éventuellement un substituant fluoro, chloro, bromo, méthyle, éthyle, propyle, isopropyle, méthoxy, éthoxy, trifluorométhyle, trifluorométhoxy,
un groupe furannyle, thiényle, pyridyle, pyrimidyle, thiazolyle ou pyrazolyle portant éventuellement dans chaque cas un substituant fluoro, chloro, bromo, méthyle ou éthyle,
un groupe phénoxy-(alkyle en C₁ à C₄) portant éventuellement un substituant fluoro, chloro, méthyle ou éthyle,
un groupe pyridyloxy-(alkyle en C₁ à C₄), pyrimidinyloxy-(alkyle en C₁ à C₄) ou thiazolyloxy-(alkyle en C₁ à C₄), chacun portant, le cas échéant, un substituant fluoro, chloro, amino, méthyle ou éthyle,
R² est un groupe alkyle en C₁ à C₁₄, alcényle en C₃ à C₆, (alkoxy en C₁ à C₄)-(alkyle en C₂ à C₆), poly-(alkoxy en C₁ à C₄) - (alkyle en C₂ à C₆) dont chacun porte éventuellement un substituant fluoro ou chloro, un groupe cycloalkyle portant éventuellement un substituant fluoro, chloro, méthyle ou éthyle,
ou bien un groupe phényle ou un groupe benzyle dont chacun porte éventuellement un substituant fluoro, chloro, nitro, méthyle, éthyle, propyle, isopropyle, méthoxy, éthoxy, trifluorométhyle, et
n a la valeur 0 ou 1.

5. Procédé de production des dérivés spirocycliques substitués de 1H-3-arylpyrrolidine-2,4-diones de formule (I) suivant la revendication 1,
**caractérisé en ce que**
(B) on obtient des composés de formule (Ib) 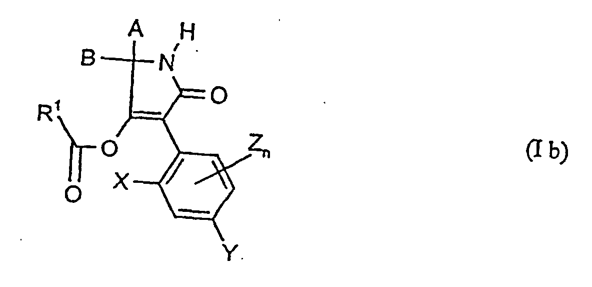 dans laquelle
A, B, X, Y, Z, R¹ et n ont la définition indiquée ci-dessus,
en faisant réagir des composés de formule (Ia) 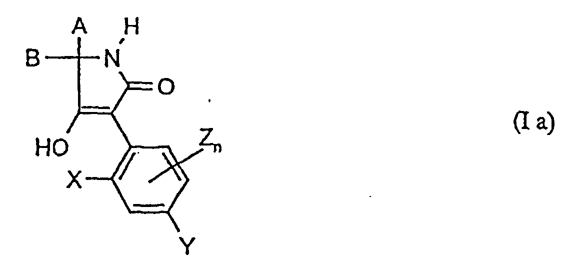 dans laquelle
A, B, X, Y, Z et n ont la définition indiquée ci-dessus,
α) avec des halogénures d'acides de formule (III) 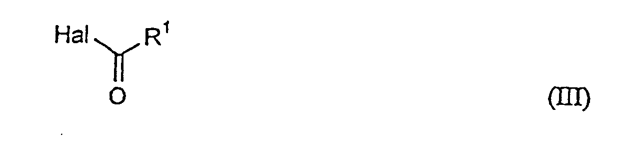 dans laquelle
R¹ a la définition indiquée ci-dessus et
Hal représente un halogène,
le cas échéant en présence d'un diluant et en présence éventuelle d'un accepteur d'acide,
ou bien
β) avec des anhydrides d'acides carboxyliques de formule (IV)
R¹-CO-O-CO-R¹ (IV)
dans laquelle
R¹ a la définition indiquée ci-dessus,
le cas échéant en présence d'un diluant et en présence éventuelle d'un accepteur d'acide,
ou bien
(C) on obtient des composés de formule (Ic-1) 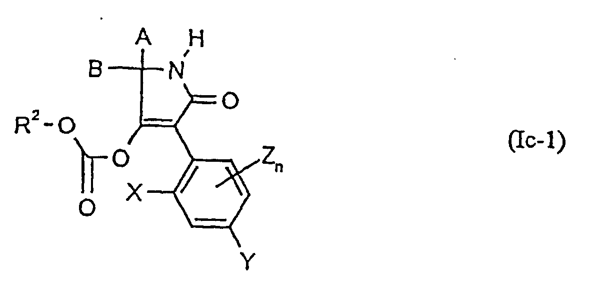 dans laquelle
A, B, X, Y, Z, R² et n ont la définition indiquée ci-dessus, en faisant réagir des composés de formule (Ia) 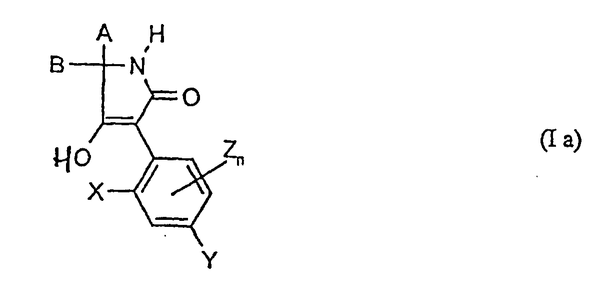 dans laquelle
A, B, X, Y, Z et n ont la définition indiquée ci-dessus,
avec des esters d'acide chloroformique de formule (V)
R²-O-CO-Cl (V)
dans laquelle
R² a la définition indiquée ci-dessus,
le cas échéant en présence d'un diluant et en présence éventuelle d'un accepteur d'acide.

6. Compositions pesticides et herbicides, **caractérisées par** une teneur en au moins un dérivé spirocyclique substitué de 1-H-3-arylpyrrolidine-2,4-dione de formule (I) suivant la revendication 1.

7. Utilisation de dérivés spirocycliques substitués de 1-H-3-arylpyrrolidine-2,4-diones de formule (I) suivant la revendication 1 pour combattre des parasites et une végétation indésirable.

8. Procédé pour combattre des parasites, **caractérisé en ce qu'**on fait agir sur les parasites, sur une végétation indésirable et/ou sur leur milieu, des dérivés spirocycliques substitués de 1-H-3-arylpyrrolidine-2,4-diones de formule (I) suivant la revendication 1.

9. Procédé de préparation de compositions pesticides et d'herbicides, **caractérisé en ce qu'**on mélange des dérivés spirocycliques substitués de 1-H-3-arylpyrrolidine-2,4-diones de formule (I) suivant la revendication 1 avec des diluants et/ou des agents tensio-actifs.
